# EUROPÄISCHE PATENTSCHRIFT

(11) **EP 2 670 736 B1**
(45) Veröffentlichungstag und Bekanntmachung des Hinweises auf die Patenterteilung: **15.03.2017**
(21) Anmeldenummer: 12702480.0
(22) Anmeldetag: 27.01.2012
(51) Int. Cl.: C07D 239/70, C07D 403/12, A61K 31/517, A61P 11/00

(54) **9-[4-(3-CHLOR-2-FLUOR-PHENYLAMINO)-7-METHOXY-CHINAZOLIN-6- YLOXY]-1,4-DIAZA-SPIRO[5.5]UNDECAN-5-ON DIMALEAT, DESSEN VERWENDUNG ALS ARZNEIMITTEL UND DESSEN HERSTELLUNG**
9-[4-(3-CHLOR-2-FLUOR-PHENYLAMINO)-7-METHOXY-QUINAZOLIN-6-YLOXY]-1,4-DIAZA-SPIRO[5.5]UNDECAN-5-ONE DIMALEATE, USE THEREOF AS A DRUG, AND PRODUCTION THEREOF
9-[4-(3-CHLORO-2-FLUORO-PHÉNYL-AMINO)-7-MÉTHOXY-QUINAZOLINE-6-YLOXY]-1,4-DIAZASPIRO[5.5]UNDÉCANE-5-ONE DIMALÉATE, PREPARATION ET UTILISATION DU 9-[4-(3-CHLORO-2-FLUORO-PHÉNYL-AMINO)-7-MÉTHOXY-QUINAZOLINE-6-YLOXY]-1,4-DIAZASPIRO[5.5]UNDÉCANE-5-ONE DIMALÉATE EN TANT QUE MEDICAMENT

(30) Priorität: 01.02.2011 EP 11152895
(43) Veröffentlichungstag der Anmeldung: 11.12.2013
(73) Patentinhaber: Boehringer Ingelheim International GmbH, 55216 Ingelheim am Rhein (DE)
(72) Erfinder: OSTERMEIER, Markus, 55216 Ingelheim Am Rhein (DE); PFRENGLE, Waldemar, 55216 Ingelheim Am Rhein (DE); HUCHLER, Guenther, 55216 Ingelheim Am Rhein (DE); SIEGER, Peter, 55216 Ingelheim Am Rhein (DE)
(74) Vertreter: Simon, Elke Anna Maria
(86) Internationale Anmeldenummer: PCT/EP2012/051298
(87) Internationale Veröffentlichungsnummer: WO 2012/104206

(56) Entgegenhaltungen:
- EP-A1- 2 289 881
- WO-A1-2009/098061
- WO-A1-2010/026029
- KUMAR L ET AL: "Salt selection in drug development", PHARMACEUTICAL TECHNOLOGY, ADVANSTAR COMMUNICATIONS,INC, US, Bd. 32, Nr. 3, 1. März 2008 (2008-03-01), Seiten 128-146, XP008127365, ISSN: 1543-2521

## Beschreibung

Gegenstand der vorliegenden Erfindung ist ein Verfahren zur stereoselektiven Herstellung der Verbindung der Formel **(I)**, 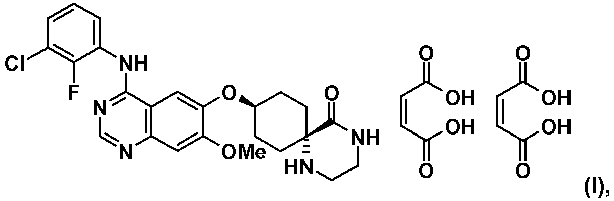 welche wertvolle pharmakologische Eigenschaften aufweist, insbesondere eine Hemmwirkung auf die durch Tyrosinkinasen vermittelte Signaltransduktion, Verfahren zur stereoselektiven Herstellung dieser Verbindung, insbesondere für die Inhalation geeignete pharmazeutische Formulierungen und deren Verwendung zur Behandlung von Krankheiten, insbesondere von Tumorerkrankungen, der benignen Prostatahyperplasie sowie von Erkrankungen der Lunge und der Atemwege

### HINTERGRUND DER ERFINDUNG

Chinazolinderivate sind aus dem Stand der Technik als Wirkstoffe beispielsweise zur Behandlung von Tumorerkrankungen als auch von Erkrankungen der Lunge und der Atemwege bekannt. Verfahren zur Herstellung von Chinazolinderivaten werden in WO03082290 und WO07068552 beschrieben. WO2009098061 offenbart die Base (Verbindung **(II)**) des erfindungsgemäßen Dimaleatsalzes (Verbindung **(I)**).

Es ist die Aufgabe der vorliegenden Erfindung ein Salz des 9-[4-(3-Chlor-2-fluorphenylamino)-7-methoxy-chinazolin-6-yloxy]-1,4-diaza-spiro[5.5]undecan-5-on bereitzustellen, welches aufgrund seiner pharmazeutischen Wirksamkeit als Tyrosin-Kinase Hemmer zur Anwendung auf therapeutischem Gebiet, d.h. zur Behandlung von pathophysiologischen Prozessen, die durch Überfunktion von Tyrosinkinasen hervorgerufen werden, geeignet ist.

Die in der vorliegenden Erfindung bereitgestellte Verbindung soll den Anforderungen an die physikalische und chemische Stabilität und an weitere Eigenschaften, wie beispielsweise kristalline Stabilität, Abwesenheit von Polymorphie und geringe Hygroskopie, insbesondere hinsichtlich Abwesenheit von Polymorphie, an einen Arzneimittelwirkstoff genügen.

Desweiteren ist es die Aufgabe der vorliegenden Erfindung ein stereoselektives Verfahren zur Herstellung der erfindungsgemäßen Verbindung bereitzustellen.

### BESCHREIBUNG DER ZEICHNUNGEN

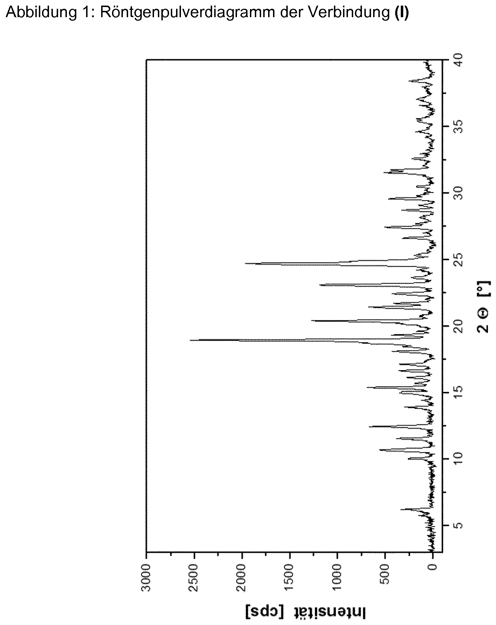

- Abbildung 1:: Röntgenpulverdiagramm der Verbindung **(I)**
- Abbildung 2:: DSC/TG-Diagramm der Verbindung **(I)**
- Abbildung 3:: Sorptionsisothermen der Verbindung **(I)**: a.) kinetischer Plot, b.) isothermer Plot

### DETAILLIERTE BESCHREIBUNG DER ERFINDUNG

Die vorliegende Erfindung löst die vorstehend genannten Aufgaben durch Bereitstellung der, insbesondere für die orale Applikation geeigneten, Verbindung der Formel **(I)**, welche hoch kristallin ist, eine geringe Hygroskopie und eine geringe Polymorphie aufweist, deren pharmazeutische Formulierung und das im Folgenden beschriebene Syntheseverfahren.

Unter kristalliner Stabilität im Rahmen der vorliegenden Erfindung ist zu verstehen, dass Röntgenpulverdiagramme der Verbindung der Formel **(I)** scharfe Reflexe mit hoher Intensität bis in den oberen 2 Θ-bereich, vorzugsweise bis zu 20 - 40 ° 2 Θ, aufweist.

Unter geringer Hygroskopie im Rahmen der vorliegenden Erfindung ist zu verstehen dass in Sorptionsexperimenten der Verbindung der Formel **(I)** eine Wasseraufnahme von kleiner als 1 % im untersuchten Luftfeuchtebereich von 10 - 90 % beobachtet wird.

Unter geringer Polymorphie im Rahmen der vorliegenden Erfindung ist zu verstehen, dass nach Umkristallisation aus verschiedenen Lösungsmitteln der Verbindung der Formel **(I)** maximal 5 andere Kristallmodifikationen, vorzugsweise maximal 3 andere Kristallmodifikationen, insbesondere bevorzugt keine anderen Kristallmodifikationen, erhalten werden.

Gegenstand der Erfindung ist ein Verfahren zur stereoselektiven Herstellung der Verbindung der Formel **(I)**, gegebenenfalls in Form ihrer Tautomeren, Solvate oder Hydrate: 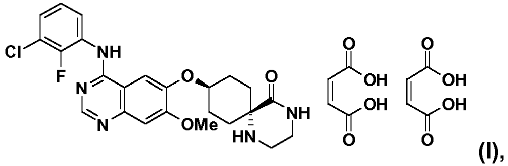 wobei das Verfahren die Reaktionsschritte **(A)** bis **(M)** umfasst, worin
**(A)** die Umsetzung von 1,4-cyclohexandion-mono-ethylenketal zu einer Verbindung der Formel **(1)** 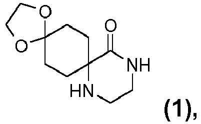
**(B)** die Umsetzung einer Verbindung der Formel **(1)** zu der Verbindung der Formel **(2)** 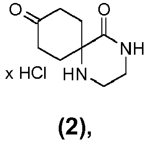
**(C)** die Umsetzung einer Verbindung der Formel **(2)** zu der Verbindung der Formel **(3)** 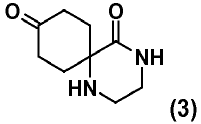
**(D)** die Umsetzung einer Verbindung der Formel **(3)** mit einem Schutzgruppenreagenz zu der Verbindung der Formel **(4)** 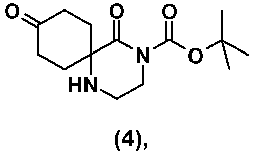
**(E)** die Reduktion einer Verbindung der Formel **(4)** zu der Verbindung der Formel **(5)** 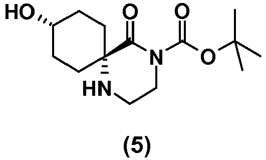
**(F)** die Umsetzung einer Verbindung der Formel **(5)** zu einer Verbindung der Formel **(6)** 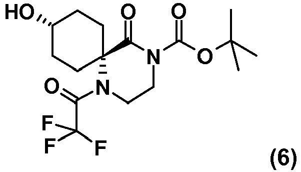
**(G)** die Umsetzung einer Verbindung der Formel **(6)** mit einer Verbindung der Formel **(13)** zu einer Verbindung der Formel **(7)** 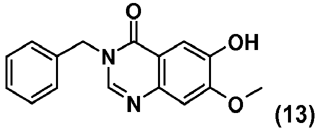 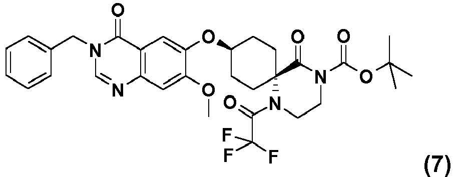
**(H)** die Umsetzung einer Verbindung der Formel **(7)** zu einer Verbindung der Formel **(8a)** oder ihrer tautomeren Form **(8b)**, 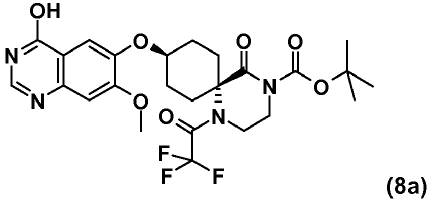 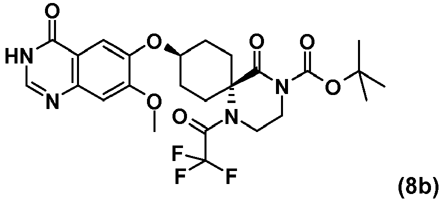
**(I)** die Chlorierung der Verbindung der Formel **(8a)** oder **(8b)** zu einer Verbindung der Formel **(9)** 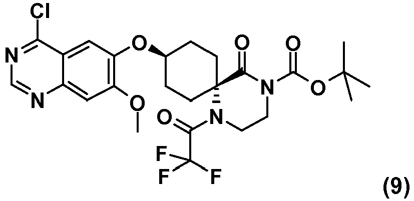
**(J) + (K)** die Umsetzung der Verbindung der Formel **(9)** mit 3-Chlor-2-fluoranilin und Abspaltung der tert-Butylcarbamat Schutzgruppe zu einer Verbindung der Formel **(11)** oder **(11A)** 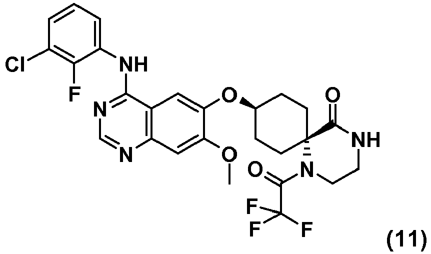 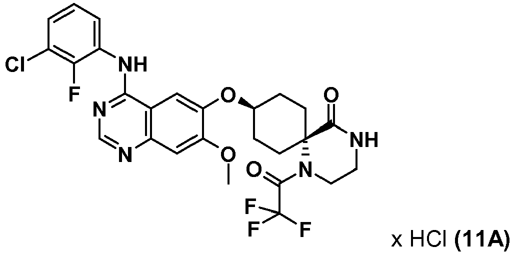
**(L)** Abspaltung der Trifluoracetyl Schutzgruppe zu der Verbindung der Formel **(II)** 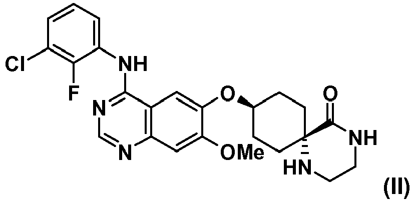
**(M)** Umsetzung der Verbindung der Formel **(II)** mit Maleinsäure zu einer Verbindung der Formel **(I)**, gegebenenfalls in Form ihrer Tautomeren, Solvate oder Hydrate, 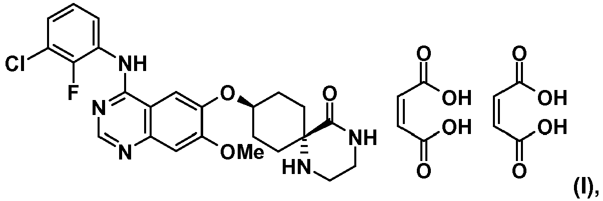 bedeutet,
   wobei die Verfahrensschritte **(A)** bis **(M)** in der genannten Reihenfolge nacheinander erfolgen.

Ein weiterer Gegenstand der Erfindung ist ein Verfahren zur stereoselektiven Herstellung der Verbindung der Formel **(I)** gegebenenfalls in Form ihrer Tautomeren: 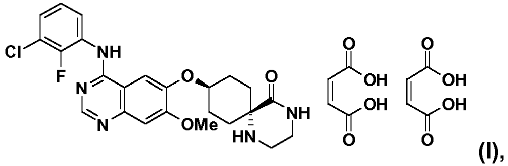 dadurch gekennzeichnet, dass das Verfahren die Reaktionsschritte **(A)** bis **(I), (N +O), (L) und (M)** umfasst, wobei
, wobei
**(A)** die Umsetzung von 1,4-cyclohexandion-mono-ethylenketal zu einer Verbindung der Formel **(1)** 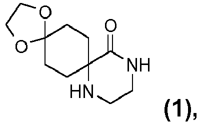
**(B)** die Umsetzung einer Verbindung der Formel **(1)** zu der Verbindung der Formel **(2)** 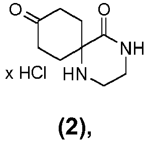
**(C)** die Umsetzung einer Verbindung der Formel **(2)** zu der Verbindung der Formel **(3)** 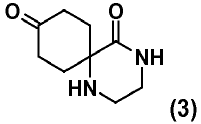
**(D)** die Umsetzung einer Verbindung der Formel **(3)** mit einem Schutzgruppenreagenz zu der Verbindung der Formel **(4)** 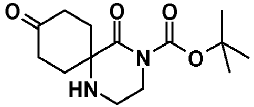 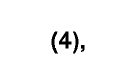
**(E)** die Reduktion einer Verbindung der Formel **(4)** zu der Verbindung der Formel **(5)** 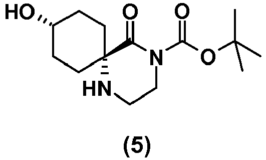
**(F)** die Umsetzung einer Verbindung der Formel **(5)**
   zu einer Verbindung der Formel **(6)** 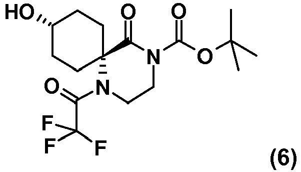
**(G)** die Umsetzung einer Verbindung der Formel **(6)** mit einer Verbindung der Formel **(13)** zu einer Verbindung der Formel **(7)** 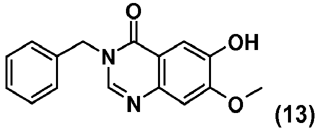 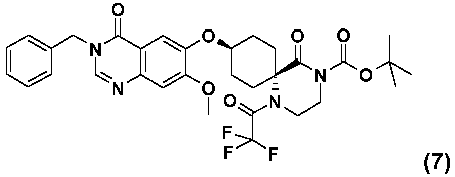
**(H)** die Umsetzung einer Verbindung der Formel **(7)** zu einer Verbindung der Formel **(8a)** oder ihrer tautomeren Form **(8b)**, 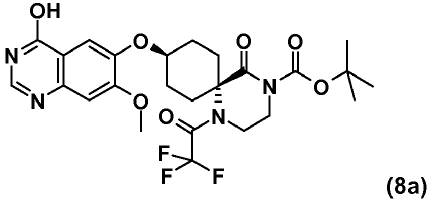 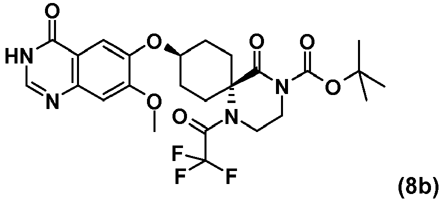
**(I)** die Chlorierung der Verbindung der Formel **(8a)** oder **(8b)** zu einer Verbindung der Formel **(9)** 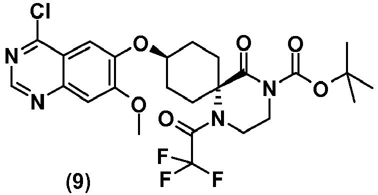
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 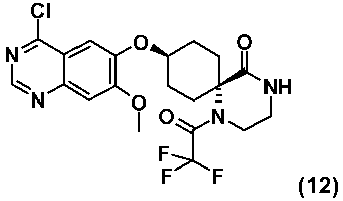
**(L)** Abspaltung der Trifluoracetyl- Schutzgruppe zu der Verbindung der Formel **(II)** 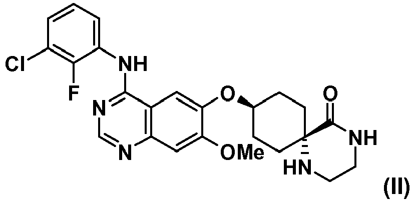
**(M)** Umsetzung der Verbindung der Formel **(II)** mit Maleinsäure zu einer Verbindung der Formel **(I)**, gegebenenfalls in Form ihrer Tautomeren 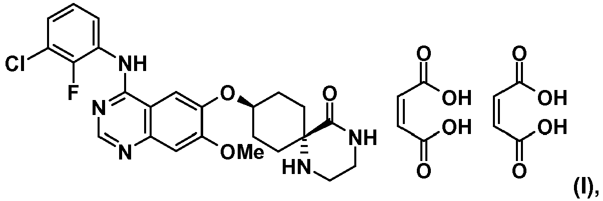 bedeutet,
   wobei die Verfahrensschritte **(A)** bis **(I), (N +O), (L) und (M)** in der genannten Reihenfolge nacheinander erfolgen.

Bevorzugt ist ein Verfahren, zur stereoselektiven Herstellung einer Verbindung der Formel **(I)**, dadurch gekennzeichnet, dass das Verfahren aus den Verfahrensschritten **(I), (J), (K), (L)**, und **(M)** oder aus den Verfahrensschritten **(I),(N), (O), (L)** und **(M)** besteht, wobei
**(I)** die Chlorierung der Verbindung der Formel **(8a)** oder **(8b)** zu einer Verbindung der Formel **(9)** 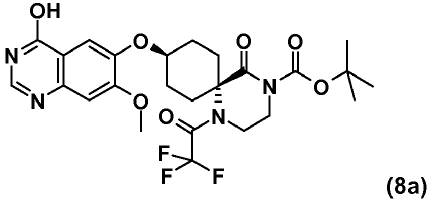 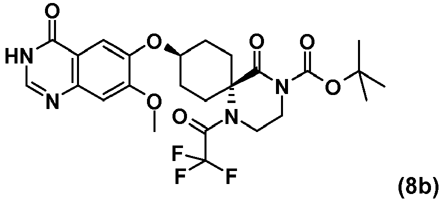 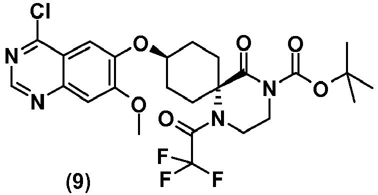
**(J) + (K)** die Umsetzung der Verbindung der Formel **(9)** mit 3-Chlor-2-fluoranilin und Abspaltung der tert-Butylcarbamat-Schutzgruppe zu einer Verbindung der Formel **(11)** oder **(11A)** 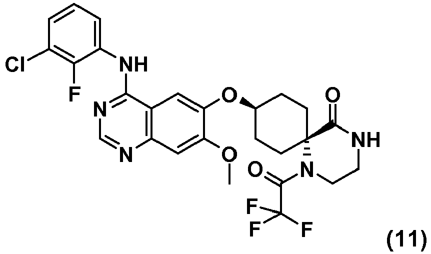 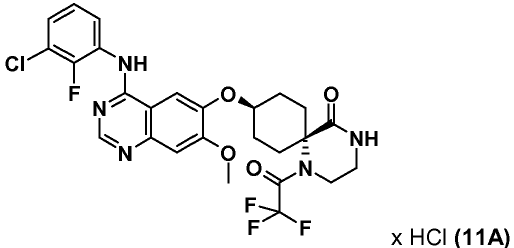
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 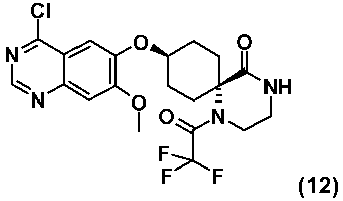
**(L)** Abspaltung der Trifluoracetyl- Schutzgruppe zu der Verbindung der Formel **(II)** 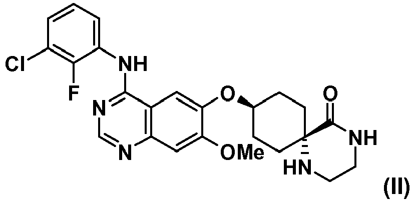
**(M)** Umsetzung der Verbindung der Formel **(II)** mit Maleinsäure zu einer Verbindung der Formel **(I)**, gegebenenfalls in Form ihrer Tautomeren 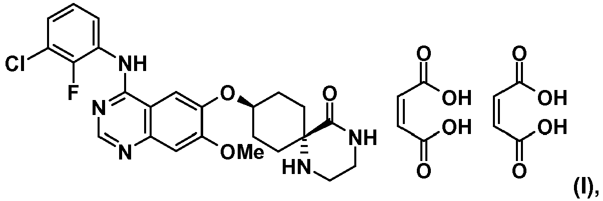 bedeutet,
   wobei die Verfahrensschritte **(I), (J), (K), (L)**, und **(M)** oder die Verfahrensschritten **(I),(N), (O), (L)** und **(M)** jeweils in der genannten Reihenfolge nacheinander erfolgen.

Weiterhin bevorzugt ist ein Verfahren zur stereoselektiven Herstellung einer Verbindung der Formel **(II)**, dadurch gekennzeichnet, dass das Verfahren aus den Verfahrensschritten **(I), (J), (K)** und **(L)**, oder aus den Verfahrensschritten **(I), (N), (O)** und **(L)** besteht, wobei
**(I)** die Chlorierung der Verbindung der Formel **(8a)** oder **(8b)** zu einer Verbindung der Formel **(9)** 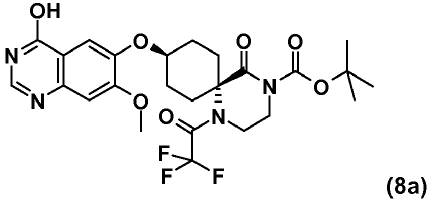 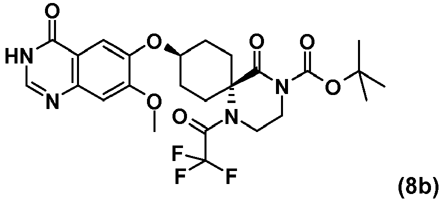 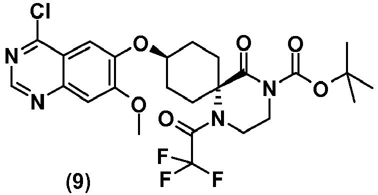
**(J) + (K)** die Umsetzung der Verbindung der Formel **(9)** mit 3-Chlor-2-fluoranilin und Abspaltung der tert-Butylcarbamat-Schutzgruppe zu einer Verbindung der Formel **(11)** oder **(11A)** 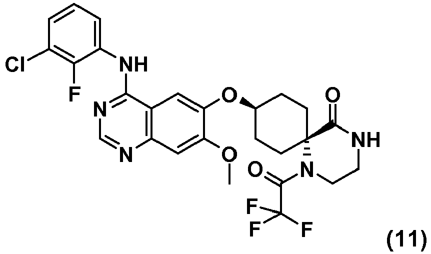 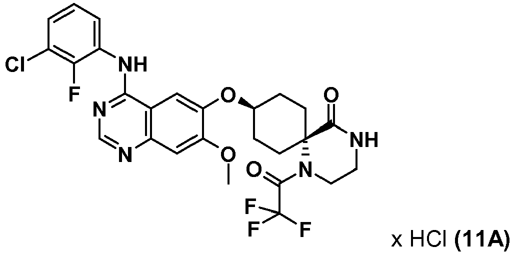
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 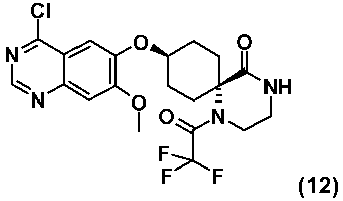
**(L)** Abspaltung der Trifluoracetyl- Schutzgruppe zu der Verbindung der Formel **(II)** 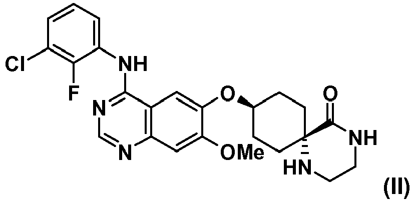 wobei die Verfahrensschritte **(I), (J), (K)** und **(L)** oder die Verfahrensschritten **(I), (N), (O)** und **(L)** jeweils in der genannten Reihenfolge nacheinander erfolgen.

Insbesondere bevorzugt ist Verfahrensschritt **(G)**, dadurch gekennzeichnet, dass
**(G)** die Umsetzung einer Verbindung der Formel **(6)** mit einer Verbindung der Formel **(13)** zu einer Verbindung der Formel **(7)** 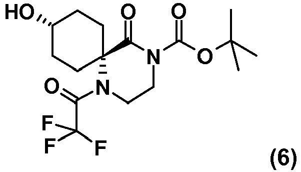 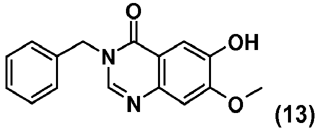 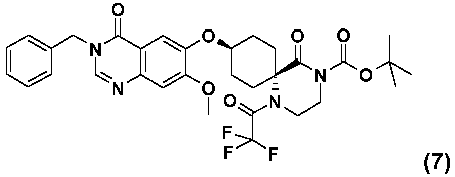 bedeutet.

Weiterhin insbesondere bevorzugt ist Verfahrensschritt **(I)**
, dadurch gekennzeichnet, dass
**(I)** die Chlorierung der Verbindung der Formel **(8a)** oder **(8b)** zu einer Verbindung der Formel **(9)** 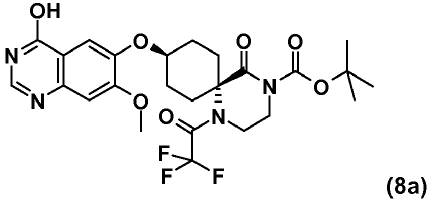 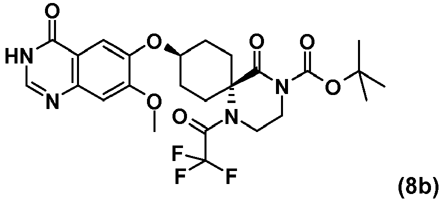 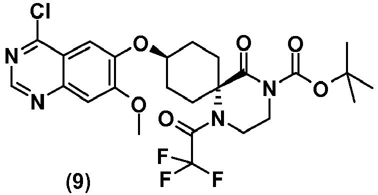 bedeutet.

Ein weiterer Gegenstand der Erfindung ist das Intermediat der Formel **(6)**, 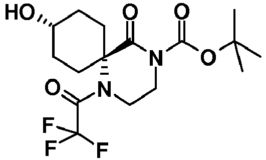 gegebenenfalls in Form ihrer Tautomeren, Solvate oder Hydrate.

Ein weiterer Gegenstand der Erfindung ist das Intermediat der Formel **(7)**, 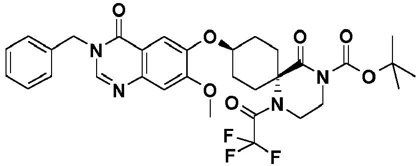 gegebenenfalls in Form ihrer Tautomeren, Solvate oder Hydrate.

Ein weiterer Gegenstand der Erfindung ist das Intermediat der Formel **(8)**, 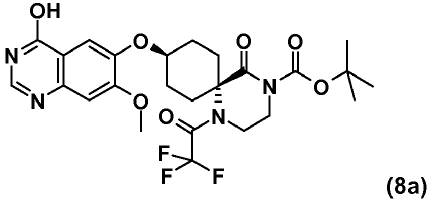 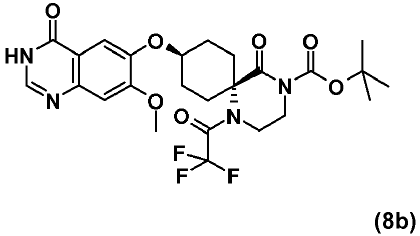 gegebenenfalls in Form ihrer Tautomeren, Solvate oder Hydrate.

Ein weiterer Gegenstand der Erfindung ist das Intermediat der Formel **(9)**, 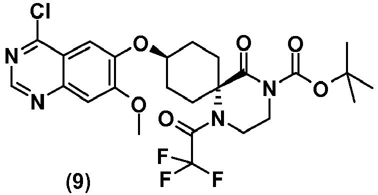 gegebenenfalls in Form ihrer Tautomeren, Solvate oder Hydrate.

Ein weiterer Gegenstand der Erfindung ist das Intermediat der Formel **(11)** oder **(11A)**, 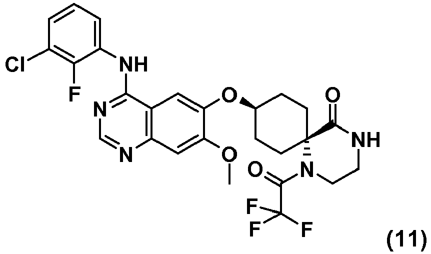 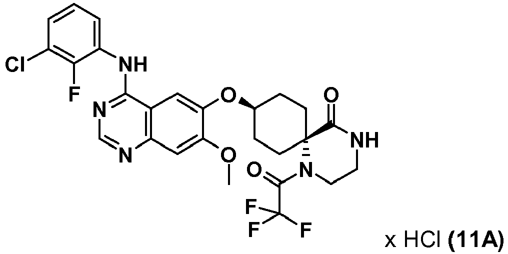 gegebenenfalls in Form ihrer Tautomeren, Solvate oder Hydrate.

In Verfahrensschritten A, C bis L und N können alternative Reagenzien ausgewählt aus der Gruppe bestehend aus den im folgenden gelisteten Reagenzien eingesetzt werden:
In Verfahrensschritt
   - A:: in Ergänzung zu Ethylendiamin und Chloroform :
   vorzugsweise Benzyltriethylammoniumchlorid /NaOH, Tetrabutylammoniumchlorid/ KOH, Benzyltriethylammoniumchlorid /KOH,₋Tetrabutylammoniumchlorid/ NaOH, insbesondere bevorzugt Benzyltriethylammoniumchlorid/ NaOH;
   - C:: vorzugsweise Alkoholatbasen ausgewählt aus der Gruppe bestehend aus NaO^{t}Bu, KO^{t}Bu und NaOEt, Carbonatbasen ausgewählt aus der Gruppe bestehend aus Cs₂CO₃, K₂CO₃, Li₂CO₃ und Na₂CO₃, insbesondere bevorzugt Natriummethylat;
   - D:: in Ergänzung zu Di-tert-butyldicarbonat und DMAP (4-(Dimethylamino)-pyridin): vorzugsweise K₂CO₃,Cs₂CO₃, Li₂CO₃ und Na₂CO₃, insbesondere bevorzugt K₂CO₃;
   - E:: vorzugsweise NaBH₄ und LiBH₄, insbesondere bevorzugt NaBH₄;
   - F:: in Ergänzung zu Trifluoressigsäureanhydrid:
   als Base vorzugsweise Triethylamin, Hünigbase, N-Methylmorpholin und N,N-Diethylanilin, insbesondere bevorzugt Triethylamin;
   - G:: in Ergänzung zu 3-Benzyl-6-hydroxy-7-methoxy-3H-quinazolin-4-one: vorzugsweise Triphenylphosphin/ Azodicarbonsäure-diisopropylester, Triphenylphosphin/Azodicarbonsäure-diethylester, Tributylphosphin/ 1,1'-(Azodicarbonyl)dipiperidin, insbesondere bevorzugt Triphenylphosphin/ Azodicarbonsäure-diisopropylester;
   - H:: Katalysatoren, vorzugsweise ausgewählt aus der Gruppe bestehend aus Pd/C und Pd(OH)₂, insbesondere bevorzugt Pd/C;
   - I:: vorzugsweise N-Chlorsuccinimid/Triphenylphosphan (in Kombination), Oxalylchlorid, Thionylchlorid, Phosphoroxychlorid, Phosphorpentachlorid, Tetrachlorkohlen-stoff/Triphenylphosphan, Dichlortriphenylphosphoran und P,P-Dichlor-phenylphosphinoxid, insbesondere bevorzugt N-Chlorsuccinimid/ Triphenylphosphan (in Kombination);
   - J+K:: in Ergänzung zu 3-Chlor-2-fluoranilin:
   vorzugsweise HCl, Methansulfonsäure, Ethansulfonsäure, p-Toluolsulfonsäure und HBr, insbesondere bevorzugt HCl;
   - L:: vorzugsweise Ethanolamin, Ammoniak und Ba(OH)₂, insbesondere bevorzugt Ethanolamin;
   - I+N:: vorzugsweise N-Chlorsuccinimid/Triphenylphosphan (in Kombination), HCl, Oxalylchlorid, Thionylchlorid, Phosphoroxychlorid, Phosphorpentachlorid, Tetrachlorkohlenstoff/Triphenylphosphan, Dichlortriphenylphosphoran, P,P-Dichlorphenylphosphinoxid, Methansulfonsäure, Ethansulfonsäure, p-Toluolsulfonsäure und HBr, insbesondere bevorzugt N-Chlorsuccinimid/Triphenylphosphan (in Kombination) und HCl.

Der Einsatz folgender Lösungsmittel ausgewählt aus der jeweils aufgeführten Gruppe wird in den oben beschriebenen Verfahrensschritten bevorzugt:
In Verfahrensschritt
   A: CH₂Cl₂, CHCl₃, THF (Tetrahydrofuran) und Dioxan;
   B: HOAc, Dioxan, H₂O und wässrige Lösungen folgender Lösungsmittel ausgewählt aus der Gruppe bestehend aus EtOH, THF, iPrOH, MeOH, NMP (N-Methyl-2-pyrrolidon) und DMF (Dimethylformamid);
   C: ACN (CH₃CN), EtOH, MeOH, iPrOH, H₂O, THF und NMP;
   D: ACN, EtOH und NMP;
   E: H₂O, MeOH, EtOH, THF und Dioxan;
   F: Me-THF, THF, Toluol, CH₂Cl₂ und Dioxan;
   G: THF, NMP, Dioxan, DMF und CH₂Cl_{2;}
   H: iPrOH, Dioxan, EtOH, MeOH, THF und NMP;
   I: Dioxan/ACN und THF/Dioxan;
   J: ACN, Dioxan, THF und EtOH;
   K: ACN, Dioxan, THF und EtOH;
   L: EtOH, MeOH, iPrOH und Dioxan;
   M: EtOH, MeOH und H₂O;
   N: Dioxan/ACN und THF/Dioxan
   O: EtOH, n-PrOH, Dioxan und NMP

Die oben beschriebenen Verfahrensschritte werden vorzugsweise in folgenden Temperaturbereichen durchgeführt:
In Verfahrensschritt:
   A: vorzugsweise -15 bis 40 °C, besonders bevorzugt 0 bis 20 °C;
   B: vorzugsweise 0 bis 100 °C, besonders bevorzugt 75 bis 100 °C;
   C: vorzugsweise 0 bis 65 °C, besonders bevorzugt 15 bis 30 °C;
   D: vorzugsweise 10 bis 80 °C, besonders bevorzugt 15 bis 35 °C;
   E: vorzugsweise 0 bis 40 °C, besonders bevorzugt 0 bis 15 °C;
   F: vorzugsweise -10 bis 60 °C, besonders bevorzugt 0 bis 35 °C;
   G: vorzugsweise 10 bis 65 °C, besonders bevorzugt 45 bis 60 °C;
   H: vorzugsweise 20 bis 85 °C, besonders bevorzugt 70 bis 85 °C;
   I: vorzugsweise 20 bis 100 °C, besonders bevorzugt 70 bis 95 °C;
   J: vorzugsweise 20 bis 100°C, besonders bevorzugt 50 bis 85 °C;
   K: vorzugsweise 20 bis 100°C, besonders bevorzugt 50 bis 85 °C;
   L: vorzugsweise 50 bis 80 °C, besonders bevorzugt 70 bis 80 °C;
   M: vorzugsweise 0 bis 75 °C, besonders bevorzugt 0 bis 70 °C.
   N: vorzugsweise 20 bis 100°C, besonders bevorzugt 50 bis 85 °C;
   O: vorzugsweise 50 bis 100°C, besonders bevorzugt 70 bis 80 °C;

Es werden vorzugsweise Schutzgruppen ausgewählt aus der Gruppe bestehend aus Benzyl, Cbz, Trifluoracetyl und Boc , insbesondere Trifluoracetyl und Boc, eingesetzt.

Die in obigen Formeln verwendete Abkürzung Boc bedeutet teriär butyl carbamat und Cbz bedeutet Benzyloxycarbonyl.

Schema 1 illustriert die erfindungsgemäße Synthese. Sämtliche Verbindungen sind in Form ihrer Basen dargestellt.

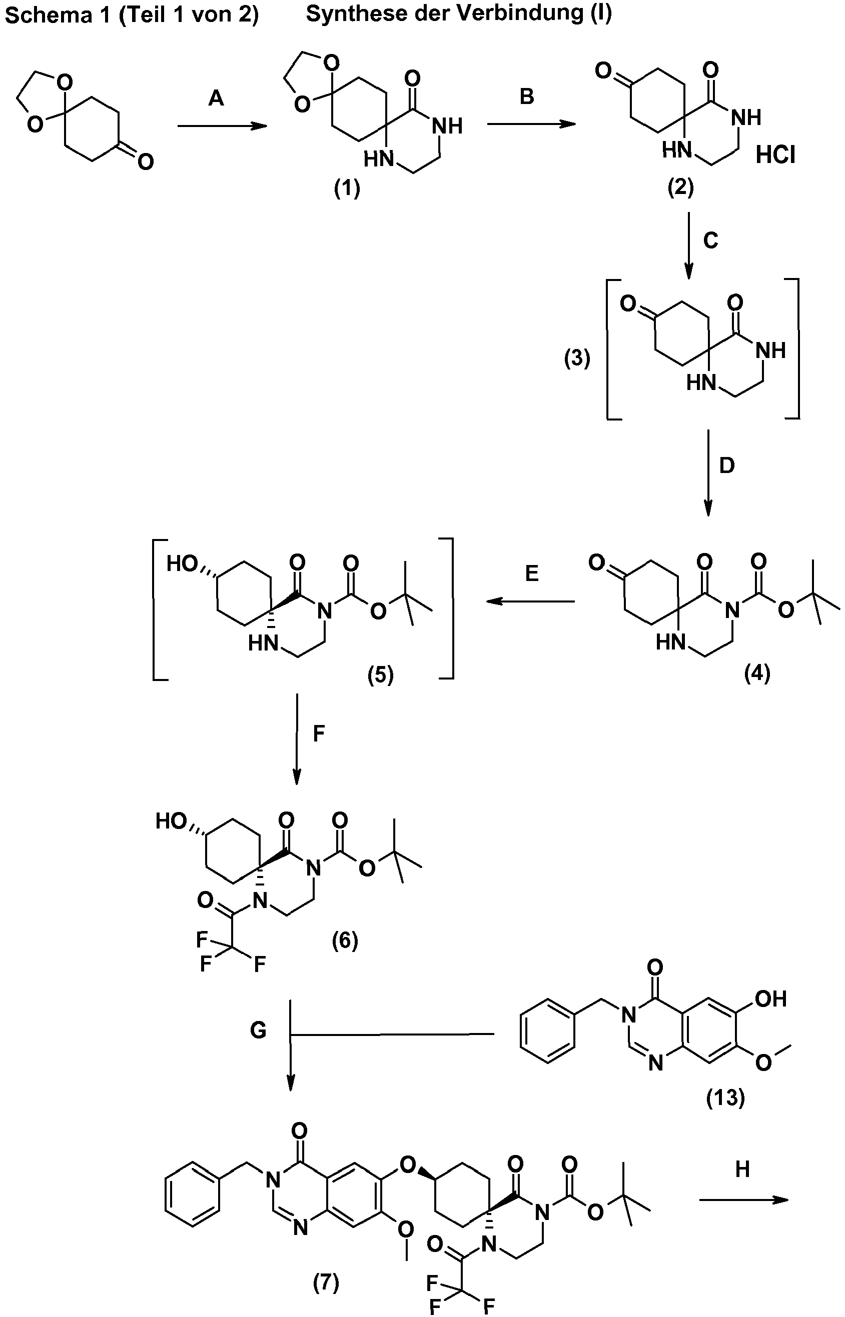

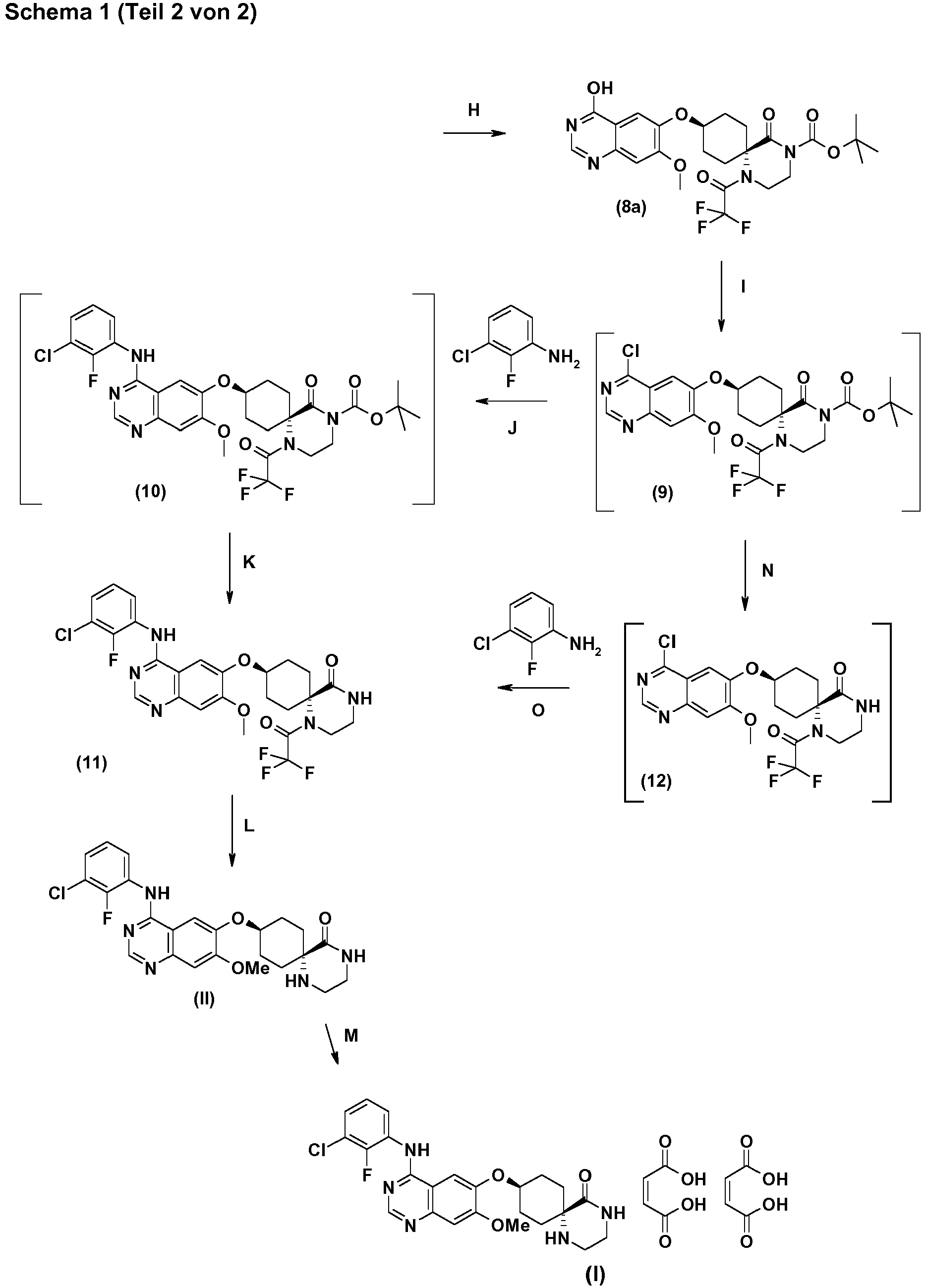

Die nachfolgenden Beispiele dienen der Illustration der exemplarisch durchgeführten Verfahren zur Herstellung der Verbindung der Formel **(I)** Diese Beispiele sind als Erläuterung der Erfindung zu verstehen, ohne selbiges auf deren Gegenstand zu beschränken.

### Beispiel 1

### 1,4-Dioxa-9,12-diaza-dispiro[4.2.5.2]pentadecan-13-on

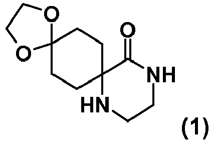

### Verfahrensschritt A

Zu einer Mischung aus 437 g Benzyltriethylammoniumchlorid und 2700 ml Ethylendiamin in 19.2 L Dichlormethan werden bei 5°C 15.1 kg 50%ige Natronlauge zugetropft. Anschließend wird eine Lösung aus 6000 g 1,4-Cyclohexandion-mono-ethylenketal und 6100 g Chloroform in 4.8 L Dichlormethan bei 5-15°C innerhalb der nächsten 4.5 h zugetropft. Der Tropftrichter wird mit 3 L Dichlormethan nachgespült. Nach 15 h werden bei 15-25°C 18 L Wasser und 39 L Dichlormethan zugegeben. Die Phasen werden getrennt und die wässrige Phase wird mit 20 L Dichlormethan extrahiert. Die vereinigten organischen Phasen werden destillativ aufkonzentriert. Nachdem 72 L Lösemittel abdestilliert sind, werden zu der Suspension 48 L Isopropanol zugegeben und danach weitere 30 L Lösemittel abdestilliert. Nach Abkühlen auf 3°C wird der Niederschlag abfiltriert und zweimal mit je 7.5 L kaltem Isopropanol nachgewaschen. Nach Trocknen bei 50°C im Vakuum erhält man 6144 g Produkt.
Massenspektrum (ESI⁺): m/z = 227 [M+H]⁺

### Beispiel 2

### 1,4-Diaza-spiro[5.5]undecane-5,9-dion

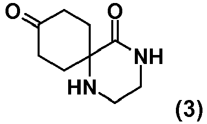

### Verfahrensschritt B

Zu 6085 g 1,4-Dioxa-9,12-diaza-dispiro[4.2.5.2]pentadecan-13-one in 25 kg Essigsäure werden bei 80-100°C 14.5 kg 4M HCl in Dioxan innerhalb von 15 min zugetropft. Der Tropftrichter wird mit 3 kg Essigsäure nachgespült. Nach 140 min wird die Suspension auf 20°C abgekühlt. Nach 2h wird der Niederschlag abfiltriert und zweimal mit je 12 L Dioxan gewaschen. Nach Trocknen bei 60°C im Vakuum erhält man 5333 g Produkt als Hydrochlorid.
Massenspektrum (ESI⁺): m/z = 183 [M+H]⁺

### Verfahrensschritt C

5200 g 1,4-Diaza-spiro[5.5]undecane-5,9-dione hydrochoride in 52 L Acetonitril werden bei RT innerhalb von 3h mit 4370 ml 30%iger Natriummethylat-Lösung in Methanol versetzt. Der Tropftrichter wird mit 1 L Methanol nachgespült. 250 g Natriumcarbonat werden zugegeben und der Ansatz für 16 h gerührt. Es werden 30 L Lösungsmittel abdestilliert und nach Zugabe von 20 L Acetonitril wird die Suspension filtriert. Der Filterkuchen wird mit 10 L Acetonitril gewaschen. Vom Filtrat werden 22 L Lösungsmittel abdestilliert und der Rückstand, welcher das Produkt enthält, wird direkt in der nächsten Stufe weiter umgesetzt.

### Beispiel 3

### 5,9-Dioxo-1,4-diaza-spiro[5.5]undecane-4-carboxylic acid tert-butyl ester

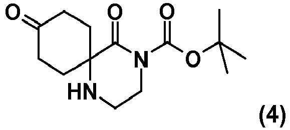

### Verfahrensschritt D

Zu dem Rückstand aus dem vorherigen Ansatz, der das 1,4-Diaza-spiro[5.5]undecane-5,9-dione enthält, werden 6573 g Kaliumcarbonat und 145 g 4-(Dimethylamino)-pyridin zugefügt. Anschließend werden innerhalb von 30 min 6487 g Di-tert-butyldicarbonat in 8 L Acetonitril zugetropft. Der Tropftrichter wird mit 2 L Acetonitril nachgespült. Nach 100 min wird der Ansatz bei 10°C auf 20 L Wasser gegeben. Es wird mit 2 L Wasser, 5 L Acetonitril und 16 L Toluol nachgespült. Nach Phasentrennung wird die organische Phase mit 10 L Toluol versetzt. Es werden 55 L Lösungsmittel abdestilliert. Nach Zugabe von 30 L Methylcyclohexan und 10 L Toluol wird mit Produkt angeimpft und die Suspension bei 20-30°C für 14h gerührt. Es werden 20 L Methylcyclohexan zugegeben und auf -5°C abgekühlt. Nach 2.5h wird der Niederschlag abfiltriert und mit 10 L Methylcyclohexan gewaschen. Nach Trocknen bei 50°C im Vakuum erhält man 5160 g Produkt.
Massenspektrum (ESI⁺): m/z = 283 [M+H]⁺

### Beispiel 4

### (cis)-9-Hydroxy-5-oxo-1,4-diaza-spiro[5.5]undecane-4-carboxylic acid tert-butyl ester

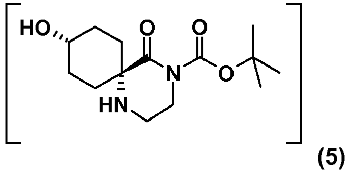

### Verfahrensschritt E

Zu einer Mischung aus 5000 g 5,9-Dioxo-1,4-diaza-spiro[5.5]undecane-4-carboxylic acid tert-butyl ester in 35 L Wasser werden bei 3°C innerhalb von 17 min 201 g Natrium-borhydrid in 5 L Wasser zugetropft. Der Tropftrichter wird mit 1.4 L Wasser nachgespült. Nach 15 min werden 30 L Methyl-Tetrahydrofuran zugegeben. Nach Zugabe von 10 L ges. Kaliumcarbonatlösung werden die Phasen getrennt und die wässrige Phase wird mit 20 L Methyl-Tetrahydrofuran extrahiert. Die vereinigten organischen Phasen werden mit 1 L ges. Kochsalz-Lsg. gewaschen. Die organische Phase wird abgetrennt und mit 27.5 L Methyl-Tetrahydrofuran verdünnt. Es werden 55 L Lösungsmittel abdestilliert. Anschließend werden 15 L Methyl-Tetrahydrofuran zugegeben und 15 L Lösungsmittel abdestilliert. Anschließend werden 20 L Methyl-Tetrahydrofuran zugegeben und 20 L Lösungsmittel abdestilliert. Anschließend werden 20 L Methyl-Tetrahydrofuran zugegeben und 20 L Lösungsmittel abdestilliert. Der Rückstand, der das Produkt enthält, wird direkt in der nächsten Stufe weiter umgesetzt.
Massenspektrum (ESI⁺): m/z = 285 [M+H]⁺

### Beispiel 5

### (cis)-9-Hydroxy-5-oxo-1-(2,2,2-trifluoro-acetyl)-1,4-diaza-spiro[5.5]undecane-4-carboxylic acid tert-butyl ester

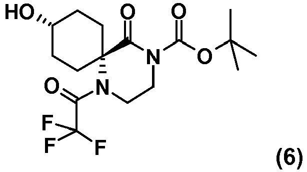

### Verfahrensschritt F

Zur Organischen Phase aus dem vorherigen Ansatz werden 11.1 L Triethylamin zugegeben. Anschließend werden bei 3-25°C 5170 ml Trifluoressigsäureanhydrid innerhalb von 30 min zugetropft. Nach 15 min werden 12.4 L Methanol zugegeben. Nach 1h werden 30 L Lösungsmittel im Vakuum abdestilliert. Anschließend werden 15.3 L Methanol zugegeben und 8 L Lösungsmittel im Vakuum abdestilliert. Es werden 12.4 L Methanol zugegeben und bei 1-10°C innerhalb von 50min 35 L Wasser zugetropft. Nach 1h bei 2°C wird der Niederschlag abzentrifugiert und mit 10 L eines 2:1-Gemisches aus Wasser und Methanol und danach noch mit 10 L Wasser gewaschen. Nach Trocknen bei 55°C im Umlufttrockenschrank erhält man 5299 g Produkt als cis-/trans-Gemisch.

Dieses Rohprodukt wird in 70 L Toluol suspendiert. Anschließend werden 500 ml Lösungsmittel abdestilliert und dann 10 L Toluol zugefügt. Die Lösung wird abgekühlt und bei 52°C wird mit Produkt angeimpft. Nach 3h bei 1°C wird der Niederschlag abzentrifugiert und mit 8 L kaltem Toluol gewaschen. Nach Trocknen bei 55°C im Vakuum erhält man 4398 g Produkt, das noch ca. 4% trans-Produkt enthält.
Massenspektrum (ESI⁺): m/z = 381 [M+H]⁺

### Beispiel 6

### (trans)-9-(3-Benzyl-7-methoxy-4-oxo-3,4-dihydro-quinazolin-6-yloxy)-5-oxo-1-(2,2,2-trifluoro-acetyl)-1,4-diaza-spiro[5.5]undecane-4-carboxylic acid tert-butyl ester

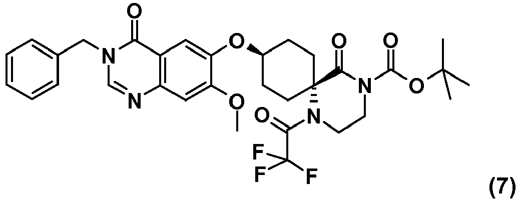

### Verfahrensschritt G

Zu einer Mischung aus 3500 g (cis)-9-Hydroxy-5-oxo-1-(2,2,2-trifluoro-acetyl)-1,4-diazaspiro[5.5]undecane-4-carboxylic acid tert-butyl ester, 2362 g 3-Benzyl-6-hydroxy-7-methoxy-3H-quinazolin-4-one und 3292 g Triphenylphosphin in 45 L Tetrahydrofuran wird bei 50-55°C innerhalb von 100 min 2471 ml Azo-dicarbonsäure-diisopropylester zugetropft. Der Tropftrichter wird mit 4 L Tetrahydrofuran nachgespült und im Vakuum werden 30 L Lösungsmittel abdestilliert. Anschließend werden, während der kontinuierlichen Zugabe von 60 L Ethanol, weitere 30 L Lösungsmittel bei Normaldruck abdestilliert. Es wird mit Produkt angeimpft und langsam auf RT abkühlen lassen. Nach 19 h wird der Niederschlag abfiltriert und mi 15 L Ethanol gewaschen. Nach Trocknen bei 50°C im Vakuum erhält man 4710 g Produkt.
Massenspektrum (ESI⁺): m/z = 645 [M+H]⁺

### Beispiel 7

### (trans)-9-(4-Hydroxy-7-methoxy-quinazolin-6-yloxy)-5-oxo-1-(2,2,2-trifluoro-acetyl)-1,4-diaza-spiro[5.5]undecane-4-carboxylic acid tert-butyl ester

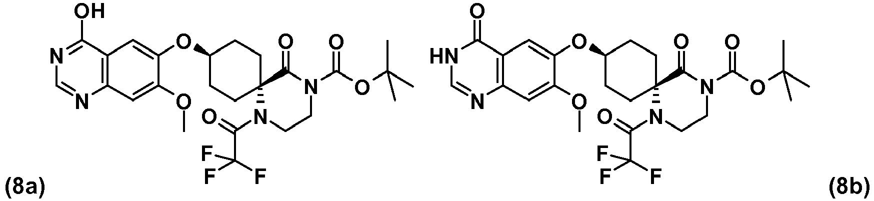

### Verfahrensschritt H

Zu einer Mischung aus 4700g (trans)-9-(3-Benzyl-7-methoxy-4-oxo-3,4-dihydro-quinazolin-6-yloxy)-5-oxo-1-(2,2,2-trifluoro-acetyl)-1,4-diaza-spiro[5.5]undecane-4-carboxylic acid tert-butyl ester in 33 L Isopropanol und 33 L Dioxan werden 470 g Palladium (10%) auf Kohle zugefügt. Nach 4 h Hydrieren bei 80°C wird bei 60°C filtriert und mit einer Mischung aus 10 L Isopropanol und 10 L Dioxan nachgewaschen. Vom Filtrat werden im Vakuum 58 L Lösungsmittel abdestilliert und 32 L tert-Butylmethylether zugefügt. Nach 2 h bei 0-5°C wird der Niederschlag abfiltriert und mit 15 L tert-Butylmethylether gewaschen. Nach Trocknen bei 50°C im Vakuum erhält man 4153 g Produkt.
Massenspektrum (ESI⁺): m/z = 555 [M+H]⁺

### Beispiel 8

### (trans)-9-(4-Chloro-7-methoxy-quinazolin-6-yloxy)-5-oxo-1-(2,2,2-trifluoro-acetyl)-1,4-diaza-spiro[5.5]undecane-4-carboxylic acid tert-butyl ester

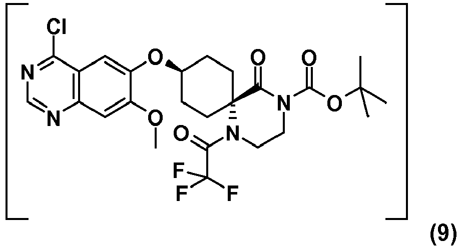

### Verfahrensschritt I

Zu einer Mischung aus 5500 g (trans)-9-(4-Hydroxy-7-methoxy-quinazolin-6-yloxy)-5-oxo-1-(2,2,2-trifluoro-acetyl)-1,4-diaza-spiro[5.5]undecane-4-carboxylic acid tert-butyl ester und 3122 g Triphenylphosphin in 24 L Dioxan werden bei 60°C 1590 g N-Chlorsuccinimid in 20 L Acetonitril innerhalb einer Minute zugegeben. Der Tropftrichter wird mit 4 L Acetonitril nachgespült und der Ansatz für 30 min auf 80-90°C aufgeheizt. Der Ansatz, der das Produkt enthält, wird direkt in die nächste Stufe eingesetzt.

### Beispiel 9

### (trans)-9-[4-(3-Chloro-2-fluoro-phenylamino)-7-methoxy-quinazolin-6-yloxy]-5-oxo-1-(2,2,2-trifluoro-acetyl)-1,4-diaza-spiro[5.5]undecane-4-carboxylic acid tert-butyl ester

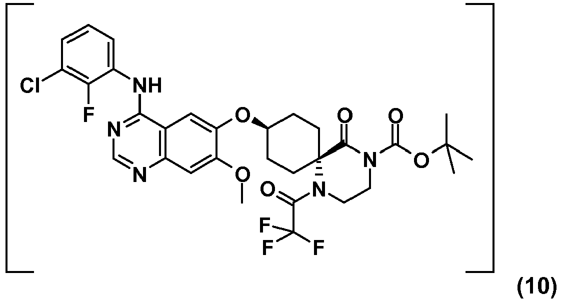

### (trans)-9-[4-(3-Chloro-2-fluoro-phenylamino)-7-methoxy-quinazolin-6-yloxy]-1-(2,2,2-trifluoro-acetyl)-1,4-diaza-spiro[5.5]undecan-5-one hydrochloride

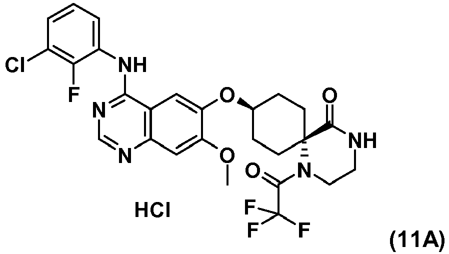

### Verfahrensschritt J+K

Nach 20 min bei 50-60°C werden zum Ansatz 1733 g 3-Chlor-2-fluoranilin zugegeben. Der Tropftrichter wird mit 2 L Acetonitril nachgespült. Anschließend werden 7.8 kg 4 M Salzsäure in Dioxan zugegeben und der Ansatz für 45 min bei 55-80°C gerührt. Nach Abkühlen auf 1°C wird der Niederschlag abfiltriert und mit 10 L Ethanol gewaschen. Der Niederschlag wird in 40 L Ethanol suspendiert und mit 290 g 3-Chlor-2-fluoranilin versetzt. Die Suspension wird für 45 min bei 70-80°C und anschließend für 13 h bei RT gerührt. Der Niederschlag wird abfiltriert und mit 10 L Ethanol gewaschen. Nach Trocknen bei 60°C im Vakuum erhält man 4853 g Produkt als Hydrochlorid.
Massenspektrum (ESI⁺): m/z = 582 [M+H]⁺

### Oder:

### Verfahrensschritt O

Eine Mischung aus 3.42g (trans)-9-(4-Chloro-7-methoxy-quinazolin-6-yloxy)-1-(2,2,2-trifluoro-acetyl)-1,4-diaza-spiro[5.5]undecan-5-one und 1.25 g 3-Chlor-2-fluoranilin in 40 ml Ethanol wird für 2 h auf 80°C erhitzt. Nach Abkühlen der Suspension auf 20°C und 16 h Rühren wird der Niederschlag abfiltriert und mit 10 mL Ethanol und 10 mL tert-Butylmethylether gewaschen. Nach Trocknen bei 100°C im Vakuum erhält man 3.28 g Produkt.

Massenspektrum (ESI⁺): m/z = 582 [M+H]⁺

### Beispiel 10

### (trans)-9-[4-(3-Chloro-2-fluoro-phenylamino)-7-methoxy-quinazolin-6-yloxy]-1,4-diazaspiro[5.5]undecan-5-one

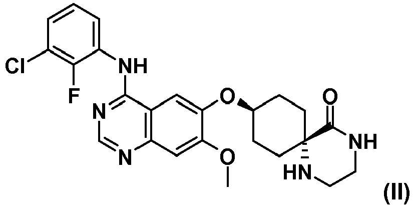

### Verfahrensschritt L

Eine Mischung aus 4700 g (trans)-9-[4-(3-Chloro-2-fluoro-phenylamino)-7-methoxy-quinazolin-6-yloxy]-1-(2,2,2-trifluoro-acetyl)-1,4-diaza-spiro[5.5]undecan-5-one und 5150 g Ethanolamin in 47 L Ethanol wird für 17 h auf 75-80°C erhitzt. Nach Abkühlen der Suspension auf 20°C wird der Niederschlag abfiltriert und mit 15 L Ethanol gewaschen. Nach Trocknen bei 60°C im Vakuum erhält man 3776 g Produkt als Mono-Ethanol-Solvat. Massenspektrum (ESI⁺): m/z = 486 [M+H]⁺
1 H NMR (400 MHz, DMSO): 9,60 (1 H, s); 8,37 (1 H, s); 7,82 (1 H, s); 7,44-7,55 (2H, m), 7,37 (1 H, s); 7,28 (1 H, t); 7,22 (1 H, s); 4,63-4,69 (1 H, m); 4.33 (1 H, t); 3,96 (3H, s); 3,41-3,49 (2H, m); 3.11-3.16 (2H, m); 2,82-2,87 (2H, m); 2,30 (1H, s); 2,14-2,23 (2H, m); 1,84-1,97 (4H, m); 1,44-1,51 (2H, m); 1.06 (3H, t).

### Beispiel 11

### (trans)-9-[4-(3-Chloro-2-fluoro-phenylamino)-7-methoxy-quinazolin-6-yloxy]-1,4-diaza-spiro[5.5]undecan-5-one Dimaleinsäure-Verbindung

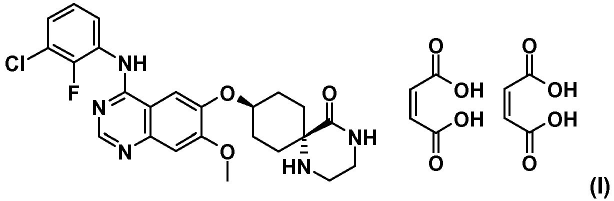

### Verfahrensschritt M

Zu 3500 g (trans)-9-[4-(3-Chloro-2-fluoro-phenylamino)-7-methoxy-quinazolin-6-yloxy]-1,4-diaza-spiro[5.5]undecan-5-one Mono-Ethanol-Solvat in 18 L Ethanol wird bei 77°C eine Lösung von 1680 g Maleinsäure in 7 L Ethanol und 7 L Wasser zugegeben. Der Tropftrichter wird mit 3 L Ethanol nachgespült. Die Lösung wird bei 66°C mit Produkt angeimpft und nach 5 min werden zu der Suspension 35 L Ethanol zugetropft. Die Suspension wird auf 20° abgekühlt und für 1 h bei dieser Temperatur gerührt. Anschließend wird auf 1°C abgekühlt und 16 h bei dieser Temperatur gerührt. Der Niederschlag wird abfiltriert und zweimal mit je 10 L Ethanol gewaschen. Nach Trocknen bei 60°C im Vakuum erhält man 4362 g Produkt.
Massenspektrum (ESI⁺): m/z = 486 [M+H]⁺
1 H NMR (400 MHz, DMSO): 8.50 (1 H, s); 8,24 (1 H, s); 7,93 (1 H, s); 7,50-7,57 (2H, m), 7,29-7.35 (1H, m); 7,27 (1H, s); 6.15 (4H, s); 4,65-4,71 (1H, m); 3,98 (3H, s); 3,45-3,51 (2H, m); 3.39-3.44 (2H, m); 2,38-2,48 (2H, m); 2,06-2,15 (2H, m); 1,83-2.02 (4H, m).

**Tabelle 1: Röntgenreflexe bis 30 ° 2 Θ inclusive Intensitäten (normalisiert) der Verbindung (I)**

| **2 Θ** | **dₕₖₗ** | **Intensität** |
|---|---|---|
| **[°]** | **[Å]** | **I/Iₒ [%]** |
| 5,76 | 15,34 | 5 |
| 6,20 | 14,25 | 12 |
| 10,00 | 8,84 | 11 |
| 10,65 | 8,30 | 22 |
| 11,52 | 7,68 | 15 |
| 12,43 | 7,11 | 26 |
| 13,89 | 6,37 | 10 |
| 14,41 | 6,14 | 4 |
| 14,95 | 5,92 | 14 |
| 15,33 | 5,77 | 27 |
| 15,68 | 5,65 | 8 |
| 16,10 | 5,50 | 10 |
| 16,61 | 5,33 | 15 |
| 17,09 | 5,18 | 14 |
| 18,06 | 4,91 | 17 |
| 18,44 | 4,81 | 13 |
| 18,92 | 4,69 | 100 |
| 19,30 | 4,60 | 17 |
| 19,60 | 4,53 | 6 |
| 20,37 | 4,36 | 51 |
| 20,70 | 4,29 | 6 |
| 21,38 | 4,15 | 26 |
| 22,41 | 3,96 | 17 |
| 23,06 | 3,85 | 49 |
| 23,64 | 3,76 | 10 |
| 24,19 | 3,68 | 7 |
| 24,67 | 3,61 | 79 |
| 25,29 | 3,52 | 8 |
| 25,68 | 3,47 | 3 |
| 26,61 | 3,35 | 13 |
| 26,94 | 3,31 | 4 |
| 27,40 | 3,25 | 21 |
| 27,67 | 3,22 | 7 |
| 28,13 | 3,17 | 6 |
| 28,68 | 3,11 | 13 |
| 29,05 | 3,07 | 6 |
| 29,55 | 3,02 | 19 |
| 29,79 | 3,00 | 7 |

### Beispiel 12

### (trans)-9-(4-Chloro-7-methoxy-quinazolin-6-yloxy)-1-(2,2,2-trifluoro-acetyl)-1,4-diazaspiro[5.5]undecan-5-one

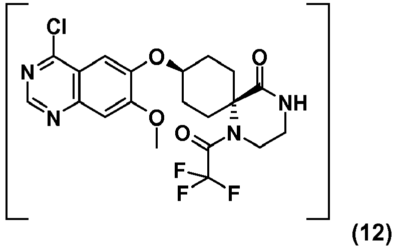

### Verfahrensschritt I+N

Zu einer Mischung aus 60 g (trans)-9-(4-Hydroxy-7-methoxy-quinazolin-6-yloxy)-5-oxo-1-(2,2,2-trifluoro-acetyl)-1,4-diaza-spiro[5.5]undecane-4-carboxylic acid tert-butyl ester und 37.5 g Triphenylphosphin in 300 mL Dioxan werden bei 60°C 19.6 g N-Chlorsuccinimid in 240 mL Acetonitril innerhalb von zwei Minuten zugegeben. Der Ansatz wird für 100 min auf 80-90°C aufgeheizt. Nach 20 min bei 50-60°C werden zum Ansatz 84 mL 4 M Salzsäure in Dioxan zugegeben und der Ansatz wird für 3 h bei 50-85°C gerührt. Nach Rühren für 17 h bei Raumtemperatur wird auf 5°C abgekühlt und der Niederschlag abfiltriert. Der Filterkuchen wird mit einem 1:1-Gemisch von Dioxan/Acetonitril und mit tert-Butylmethylether gewaschen. Nach Trocknen bei 50°C erhält man 40 g Produkt.
Massenspektrum (ESI⁺): m/z = 473 [M+H]⁺

### Erstellung von Daten

Zur Erstellung der im Anhang aufgeführten Daten werden folgende Geräte und Versuchsbedingungen verwendet:

### Röntgenpulverdiffraktometer

STOE Stadi P Röntgenpulverdiffraktometer mit einem ortsempfindlichen Detektor im Transmissionmodus mit einem gebogenen Germanium (111) Primärmonochromator; verwendete Wellenlänge: CuK_{α□} mit λ = 1.540598 Å; Betrieb der Röntgenröhre mit 40 kV, 40 mA; 2 Θ - Bereich: 3 - 40°

### Thermoanalyseausrüstung

Ein DSC 822 der Fa. Mettler Toldeo wird verwendet. Die folgenden Meßparameter werden angewendet: Heizrate: 10 K/min; Tiegeltyp: gelochte Aluminiumtiegel; Atmosphäre: N₂, 80 ml/min Flußrate; typische Einwaagen: 3 -10 mg.

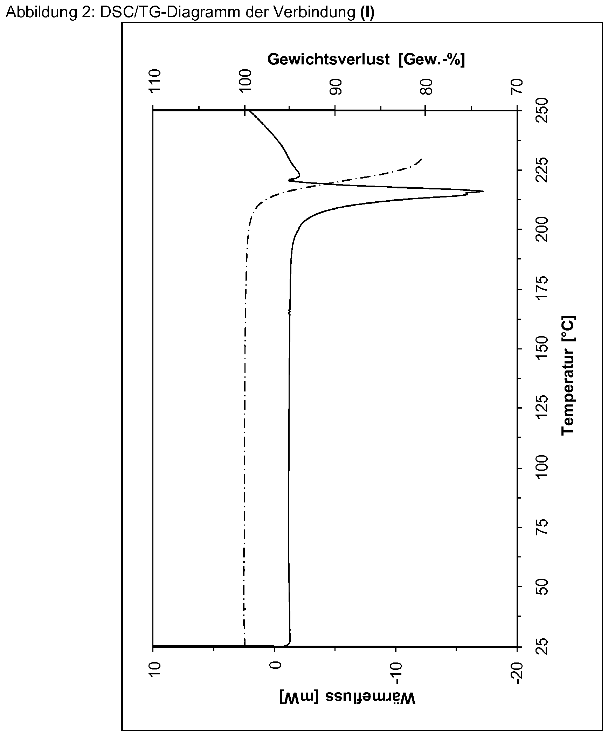

Ein TGA/SDTA 851 der Fa. Mettler Toledo, welches mit einem Nicolet FT-IR 4700 Spektrometer (zur Analyse der flüchtigen Anteile) gekoppelt ist, wird eingesetzt. Die folgenden Meßparameter werden angewendet: Heizrate: 10 K/min; Tiegeltyp: offene Aluminiumoxid Tiegel; Atmosphäre: N₂, 20 ml/min Flußrate typische Einwaagen: 15 - 25 mg.
Der Schmelzpunkt der Verbindung **(I)** kann aus dem DSC/TG-Diagramm der Abbildung 2 im Anhang entnommen werden.

### Ausrüstung für Wassersorptionsuntersuchungen

Ein DVS-1 der Fa. Surface Measurement Systems (= SMS) wird eingesetzt um das hygroskopische Verhalten zu untersuchen: folgende Feuchteprofile werden angewendet: von 10 - 90 % r.F. in Stufen von 10 %, Sorptions- so wie auch Desorptionsprofile werden aufgenommen, typische Einwaagen: 10 - 20 mg

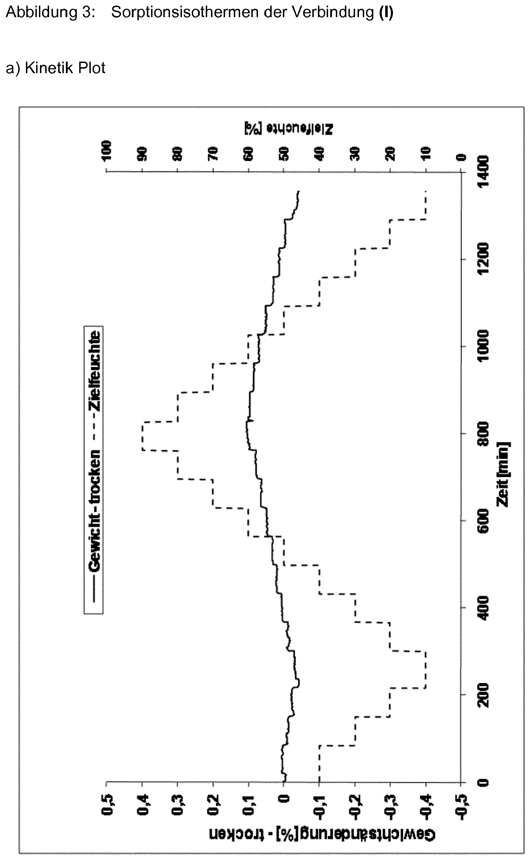

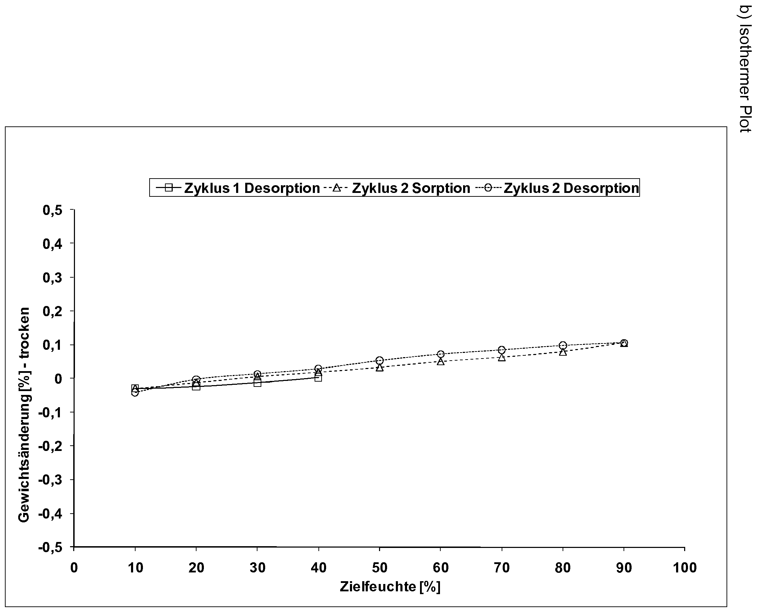

Die entsprechende Diagramme (Kinetik - und Isotherm - Plot) der verschiedenen Formen sind in den Abbildungen 3 a) und b) gezeigt.

### Biologischer Test

Die biologischen Eigenschaften der Verbindung **(I)** werden beispielsweise wie folgt geprüft:
Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Eine murine hämatopoetische Zelllinie wird derart genetisch verändert, dass sie funktionellen humanen EGF-R exprimiert. Die Proliferation dieser Zelllinie kann daher durch EGF stimuliert werden.

Der Test wird wie folgt durchgeführt:
Die Zellen werden in RPMI/1640 Medium kultiviert. Die Proliferation wird mit 20 ng/ml humanem EGF (Promega) stimuliert. Zur Untersuchung der inhibitorischen Aktivität der erfindungsgemäßen Verbindungen werden diese Verbindungen in 100% Dimethylsulfoxid (DMSO) gelöst und in verschiedenen Verdünnungen den Kulturen zugefügt, wobei die maximale DMSO Konzentration 1% beträgt. Die Kulturen werden für 48 Stunden bei 37°C inkubiert.

Zur Bestimmung der inhibitorischen Aktivität der erfindungsgemäßen Verbindung **(I)** wird die relative Zellzahl mit dem Cell Titer 96TM AQueous Non-Radioactive Cell Proliferation Assay (Promega) in O.D. Einheiten gemessen. Die relative Zellzahl wird in Prozent der Kontrolle berechnet und die Wirkstoffkonzentration, die die Proliferation der Zellen zu 50% hemmt (IC50), abgeleitet.

**Tabelle 2**

| **Verbindung** | **Hemmung der EGFR-abhängigen Proliferation IC₅₀ [nM]** |
|---|---|
| **(I)** | 4 |

### Indikationen

Wie gefunden wurde, zeichnet sich die Verbindung der Formel **(I)**, durch vielfältige Anwendungsmöglichkeiten auf therapeutischem Gebiet aus. Hervorzuheben sind solche Anwendungsmöglichkeiten, für welche die erfindungsgemäße Verbindung der Formel **(I)**, aufgrund ihrer pharmazeutischen Wirksamkeit als Tyrosinkinase-Hemmer bevorzugt zur Anwendung gelangen kann.

Die erfindungsgemäße Verbindung der allgemeinen Formel **(I)** hemmt somit die Signaltransduktion durch Tyrosinkinasen, wie am Beispiel des humanen EGF-Rezeptors gezeigt wurde, und ist daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuro-epithelialen Ursprungs, Metastasierung sowie die abnorme Proliferation vaskulärer Endothelzellen (Neoangiogenese).

Die erfindungsgemäße Verbindung **(I)** ist auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nichtallergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindung **(I)** ist auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-DarmTrakt oder wie sie bei Erkrankungen des Magen-Darm-Traktes, die mit einer vermehrten Sekretion einhergehen, vorkommen, wie M. Menetrier, sezernierende Adenome und Proteinverlustsyndrome.

Außerdem kann die Verbindung **(I)** zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

### Kombinationen

Die Verbindung der Formel **(I)**, kann allein oder in Kombination mit anderen Wirkstoffen zur Anwendung gelangen. Diese Kombinationen können entweder simultan oder sequentiell verabreicht werden. Gegebenenfalls kann die Verbindung der Formel **(I)** auch in Kombination mit **W** eingesetzt werden, worin **W** einen pharmakologisch, aktiven Wirkstoff darstellt und (beispielsweise) ausgewählt ist, aus der Gruppe bestehend aus Betamimetika, Anticholinergika, Corticosteroiden, PDE4-Inhibitoren, LTD4-Rezeptor (CysLT1, CysLT2, CysLT3) Antagonisten, LTB4-Rezeptor (BLT1, BLT2) Antagonisten, Inhibitoren von MAP Kinasen wie zum Beispiel p38, ERK1, ERK2, JNK1, JNK2, JNK3 oder SAP, Bradykinin (BK1, BK2) Rezeptor Antagonisten, Endothelin Rezeptor Antagonisten, CXCR1 und/ oder CXCR2 Rezeptor Antagonisten, und Substanzen gegen Husten. Weiterhin können zwei- oder dreifach Kombinationen von **W** mit den Verbindungen der Formel **(I)** kombiniert werden. Beispielhaft genannte Kombinationen von **W** mit den Verbindung der Formel 1 wären:
- **W** stellt ein Betamimetikum dar, kombiniert mit einem Anticholinergikum, Corticosteroid, PDE4-Inhibitor, EGFR-Hemmer oder LTD4-Rezeptor Antagonisten,
- **W** stellt ein Anticholinergikum dar, kombiniert mit einem Betamimetikum, Corticosteroid, PDE4-Inhibitor, EGFR-Hemmen oder LTD4-Rezeptor Antagonisten,
- **W** stellt ein Corticosteroid dar, kombiniert mit einem PDE4-Inhibitor, EGFR-Hemmer oder LTD4-Rezeptor Antagonist
- **W** stellt ein PDE4-Inhibitor dar, kombiniert mit einem EGFR-Hemmer oder LTD4- Rezeptor Antagonist
- **W** stellt ein EGFR-Hemmer dar, kombiniert mit einem Anticholinergikum.

Als Betamimetika gelangen hierbei vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol, Zinterol and 6-Hydroxy-8-{1-hydroxy-2-[2-(4-methoxy-phenyl)-1,1-dimethyl-ethylamino]-ethyl}-4H-benzo[1,4]oxazin-3-on, 8-{2-[2-(2,4-Difluor-phenyl)-1,1-dimethyl-ethylamino]-1-hydroxyethyl}-6-hydroxy-4H-benzo[1,4]oxazin-3-on, 8-{2-[2-(3,5-Difluor-phenyl)-1,1-dimethylethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 8-{2-[2-(4-Fluor-phenyl)-1,1-dimethyl-ethylamino]-1-hydroxy-ethyl}-6-hydroxy-4H-benzo[1,4]oxazin-3-on, N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid, N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid, N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid, N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid, 8-{2-[1,1-Dimethyl-3-(2-oxo-2,3-dihydro-benzoimidazol-1-yl)-propylamino]-1-hydroxy-ethyl}-6-hydroxy-4H-benzo[1,4]oxazin-3-on, 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on, 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on, 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on, N-[2-Hydroxy-5-((1R)-1-hydroxy-2-{2-[4-(2-hydroxy-2-phenyl-ethylamino)-phenyl]-ethylamino}-ethyl)-phenyl]-formamid, 8-Hydroxy-5-((1R)-1-hydroxy-2-{2-[4-(6-methoxy-biphenyl-3-ylamino)-phenyl]-ethylamino}-ethyl)-1H-chinolin-2-on, 8-Hydroxy-5-[(1R)-1-hydroxy-2-(6-phenethylamino-hexylamino)-ethyl]-1H-chinolin-2-on, 5-[(1R)-2-(2-{4-[4-(2-Amino-2-methyl-propoxy)-phenylamino]-phenyl}-ethylamino)-1-hydroxy-ethyl]-8-hydroxy-1H-chinolin-2-on, [3-(4-{6-[(2R)-2-Hydroxy-2-(4-hydroxy-3-hydroxymethyl-phenyl)-ethylamino]-hexyloxy}-butyl)-5-methyl-phenyl]-harnstoff, 4-((1R)-2-{6-[2-(2,6-Dichlorbenzyloxy)-ethoxy]-hexylamino}-1-hydroxy-ethyl)-2-hydroxymethyl-phenol, 3-(4-{6-[(2R)-2-Hydroxy-2-(4-hydroxy-3-hydroxymethyl-phenyl)-ethylamino]-hexyloxy}-butyl)-benzenesulfonamid, 3-(3-{7-[(2R)-2-Hydroxy-2-(4-hydroxy-3-hydroxymethyl-phenyl)-ethylamino]-heptyloxy}-propyl)-benzenesulfonamid, 4-((1R)-2-{6-[4-(3-Cyclopentanesulfonyl-phenyl)-butoxy]-hexylamino}-1-hydroxy-ethyl)-2-hydroxymethylphenol, *N*-1-Adamantanyl-2-{3-[(2R)-2-({(2R)-2-hydroxy-2-[4-hydroxy-3-(hydroxymethyl)phenyl]ethyl}amino)propyl]phenyl}acetamid, (1R)-5-{2-[6-(2,2-Difluor-2-phenyl-ethoxy)-hexylamino]-1-hydroxy-ethyl}-8-hydroxy-1H-chinolin-2-on, (R,S)-4-(2-{[6-(2,2-Difluor-4-phenylbutoxy)hexyl]amino}-1-hydroxy-ethyl)-2-(hydroxymethyl)phenol , (R,S)-4-(2-{[6-(2,2-Difluor-2-phenylethoxy)hexyl]amino}-1-hydroxy-ethyl)-2-(hydroxymethyl)phenol, (R,S)-4-(2-{[4,4-Difluor-6-(4-phenylbutoxy)hexyl]amino}-1-hydroxy-ethyl)-2- (hydroxymethyl)phenol, (R,S)-4-(2-{[6-(4,4-Difluor-4-phenylbutoxy)hexyl]amino}-1-hydroxy-ethyl)-2-(hydroxymethyl)phenol, (R,S)-5-(2-{[6-(2,2-Difluor-2-phenylethoxy)hexyl]amino}-1-hydroxy-ethyl)-8-hydroxychinolin-2(1 H)-on, (R,S)-[2-({6-[2,2-Difluor-2-(3-methylphenyl)ethoxy]hexyl}amino)-1-hydroxyethyl]-2-(hydroxymethyl)phenol, 4-(1R)-2-{[6-(2,2-Difluor-2-phenylethoxy)hexyl]amino}-1-hydroxyethyl)-2-(hydroxymethyl)phenol, (R,S)-2-(Hydroxymethyl)-4-(1-hydroxy-2-{[4,4,5l5-tetrafluor-6-(3-phenylpropoxy)hexyl]amino}ethyl)phenol, (R,S)-[5-(2-{[6-(2,2-Difluor-2-phenylethoxy)hexyl]amino}-1-hydroxy-ethyl)-2- hydroxyphenyl]formamid, (R,S)-4-[2-({6-[2-(3-Bromophenyl)-2,2-difluoroethoxy]hexyl}amino)-1-hydroxyethyl]-2-(hydroxymethyl)phenol, (R,S)-N-[3-(1,1-Difluor-2-{[6-({2-hydroxy-2-[4-hydroxy-3-(hydroxymethyl)phenyl]ethyl}amino)hexyl]oxy}ethyl)phenyl]-harnstoff, 3-[3-(1,1-Difluor-2-{[6-({2-hydroxy-2-[4-hydroxy-3-(hydroxymethyl)phenyl]ethyl}-amino)hexyl]oxy}ethyl)phenyl]imidazolidin-2,4-dion, (R,S)-4-[2-({6-[2,2-Difluor-2-(3-methoxyphenyl)ethoxy]hexyl}amino)-1-hydroxyethyl]-2-(hydroxymethyl)phenol, 5-((1R)-2-{[6-(2,2-Difluor-2-phenylethoxy)hexyl]amino}-1-hydroxyethyl)-8-hydroxychinolin-2(1H)-on, 4-((1R)-2-{[4,4-Difluor-6-(4-phenylbutoxy)hexyl]amino}-1-hydroxy-ethyl)-2-(hydroxymethyl)phenol, (R,S)-4-(2-{[6-(3,3-Difluor-3-phenylpropoxy)hexyl]amino}-1-hydroxy-ethyl)-2-(hydroxymethyl)phenol, (R,S)-(2-{[6-(2,2-Difluor-2-phenylethoxy)-4,4-difluorohexyl]amino}-1-hydroxyethyl)-2-(hydroxymethyl)phenol, (R,S)-4-(2-{[6-(2,2-Difluor-3-phenylpropoxy)hexyl]amino}-1-hydroxy ethyl)-2- (hydroxymethyl)phenol, 3-[2-(3-Chlorphenyl)-ethoxy]-N-(2-diethylamino-ethyl)-N-{2-[2-(4-hydroxy-2-oxo-2,3-dihydrobenzothiazol-7-yl)-ethylamino]-ethyl}-propionamid, N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid 7-[2-(2-{3-[2-(2-Chlor-phenyl)-ethylamino]-propylsulfanyl}-ethylamino)-1-hydroxy-ethyl]-4-hydroxy-3H-benzothiazol-2-on,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze . In den vorstehend genannten Salzen stellen die Kationen die pharmakologisch aktiven Bestandteile dar. Als Anionen X-können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt. Weiterhin genannte Verbindungen sind:
2,2-Diphenylpropionsäuretropenolester-Methobromid, 2,2-Diphenylpropionsäurescopinester-Methobromid, 2-Fluor-2,2-Diphenylessigsäurescopinester-Methobromid, 2-Fluor-2,2-Diphenylessigsäuretropenolester-Methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester-Methobromid, 9-Fluorfluoren-9-carbonsäuretropenolester-Methobromid, 9-Hydroxy-fluoren-9-carbonsäurescopinester-Methobromid, 9-Fluor-fluoren-9-carbonsäurescopinester-Methobromid, 9-Methyl-fluoren-9-carbonsäuretropenolester-Methobromid, 9-Methylfluoren-9-carbonsäurescopinester-Methobromid, Benzilsäurecyclopropyltropinester-Methobromid, 2,2-Diphenylpropionsäurecyclopropyltropinester-Methobromid, 9-Hydroxyxanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester-Methobromid, 9-Hydroxy-xanthen-9-carbonsäuretropenolester-Methobromid, 9-Hydroxy-xanthen-9-carbonsäurescopinester-Methobromid, 9-Methyl-xanthen-9-carbonsäuretropenolester-Methobromid, 9-Methylxanthen-9-carbonsäurescopinester-Methobromid, 9-Ethyl-xanthen-9-carbonsäuretropenolester-Methobromid, 9-Difluormethyl-xanthen-9-carbonsäuretropenolester-Methobromid, 9-Hydroxymethyl-xanthen-9-carbonsäurescopinester-Methobromid. Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻genannten Bedeutungen haben kann.

Als Corticosteroide gelangen hierbei vorzugsweise Verbindungen zur Anwendung, die ausgewählt sind aus der Gruppe bestehend aus: Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, Tipredane und Pregna-1,4-diene-3,20-dione, 6-fluoro-11-hydroxy-16,17-[(1-methylethylidene) bis(oxy)]-21-[[4-[(nitrooxy)methyl]benzoyl]oxy]-, (6-alpha,11-beta,16-alpha)- (9Cl) (NCX-1024), 16,17-butylidenedioxy-6,9-difluoro-11-hydroxy-17-(methylthio)androst-4-en-3-one (RPR-106541), 6,9-Difluor-17-[(2-furanylcarbonyl)oxy]-11-hydroxy-16-methyl-3-oxo-androsta-1,4-dien-17-carbothionsäure (S)-fluoromethylester, 6,9-Difluor-11-hydroxy-16-methyl-3-oxo-17-propionyloxy-androsta-1,4-dien-17-carbothionsäure (S)-(2-oxo-tetrahydro-furan-3S-yl)ester, 6-alpha, 9-alpha-difluoro-11-beta-hydroxy-16alpha-methyl-3-oxo-17alpha-(2,2,3,3-tetramethylcyclopropylcarbonyl)oxy-androsta-1,4-diene-17beta-carbonsäure cyanomethyl ester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast Pumafentrin , Lirimilast, Apremilast, Arofyllin, Atizoram, Oglemilast, Tetomilast, und 5-[(N-(2,5-dichloro-3-pyridinyl)-carboxamid]-8-methoxy-Chinolin (D-4418), N-(3,5-dichloro-1-oxido-4-pyridinyl)-carboxamid]-8-methoxy-2-(trifluoromethyl)-Chinolin (D-4396 (Sch-351591)), N-(3,5-dichloropyrid-4-yl)-[1-(4-fluorobenzyl)-5-hydroxy-indol-3-yl]glyoxylsäureamid (AWD-12-281 (GW-842470)), , 9-[(2-fluorophenyl)methyl]-N-methyl-2-(trifluoromethyl)-9H-Purin-6-amine (NCS-613), 4-[(2R)-2-[3-(cyclopentyloxy)-4-methoxyphenyl]-2-phenylethyl]-Pyridine (CDP-840), N-[(3R)-3,4,6,7-tetrahydro-9-methyl-4-oxo-1-phenylpyrrolo[3,2,1-jk][1,4]benzodiazepin-3-yl]-4-Pyridinecarboxamide (PD-168787), 4-[6,7-diethoxy-2,3-bis(hydroxymethyl)-1-naphthalenyl]-1-(2-methoxyethyl)-2(1H)-Pyridinone (T-440), 2-[4-[6,7-diethoxy-2,3-bis(hydroxymethyl)-1-naphthalenyl]-2-pyridinyl]-4-(3-pyridinyl)-1(2H)-Phthalazinone (T-2585), (3-(3-cyclopenyloxy-4-methoxybenzyl)-6-ethylamino-8-isopropyl-3H-purine (V-11294A), beta-[3-(cyclopentyloxy)-4-methoxyphenyl]-1,3-dihydro-1,3-dioxo-2H-Isoindole-2-propanamid (CDC-801), Imidazo[1,5-a]pyrido[3,2-e]pyrazin-6(5H)-one, 9-ethyl-2-methoxy-7-methyl-5-propyl- (D-22888)5-[3-(cyclopentyloxy)-4-methoxyphenyl]-3-[(3-methylphenyl)methyl]-, (3S,5S)-2-Piperidinon (HT-0712), 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141) N-(3,5-Dichloro-1-oxo-pyridin-4-yl)-4-difluormethoxy-3-cyclopropylmethoxybenzamid, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Brombenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluormethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9*H*-pyrazolo[3,4-c]-1,2,4-triazolo[4,3-a]pyridin, 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTB4-Rezeptor Antagonisten gelangen hierbei vorzugsweise Verbindungen zur Anwendung wie z.B. Amebulant (=[[4-[[3-[[4-[1-(4-hydroxyphenyl)-1-methylethyl]phenoxy]methyl]phenyl]methoxy]phenyl]iminomethyl]-Carbaminsäure-ethyl ester),
gegebenenfalls in Form ihrer Racemate, Enantiomere, Diastereomere und gegebenenfalls in Form ihrer pharmakologisch verträglichen Säureadditionssalze, Solvate, Prodrugs oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast und (E)-8-[2-[4-[4-(4-Fluorophenyl)butoxy]phenyl]ethenyl]-2-(1H-tetrazol-5-yl)-4H-1-benzopyran-4-one (MEN-91507), 4-[6-Acetyl-3-[3-(4-acetyl-3-hydroxy-2-propylphenylthio)propoxy]-2-propylphenoxy]-buttersäure (MN-001), 1-(((R)-(3-(2-(6,7-Difluor-2-chinolinyl)ethenyl)phenyl)-3-(2-(2-hydroxy-2-propyl)phenyl)thio)methylcyclopropan-essigsäure,, 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure, [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als MAP Kinase Inhibitoren gelangen hierbei vorzugsweise Verbindungen zur Anwendung, die ausgewählt sind aus der Gruppe bestehend aus:
Bentamapimod (AS-602801), Doramapimod (BIRB-796), 5-Carbamoylindole (SD-169),, 6-[(aminocarbonyl)(2,6-difluorophenyl)amino]-2-(2,4-difluorophenyl)-3-Pyridinecarboxamide (VX-702), alpha-[2-[[2-(3-pyridinyl)ethyl]amino]-4-pyrimidinyl]-2-Benzothiazoleacetonitrile (AS-601245), 9,12-Epoxy-1H-diindolo[1,2,3-fg:3',2',1'-kl]pyrrolo[3,4-i][1,6]benzodiazocine-10-Carboxylsäure (CEP-1347), 4-[3-(4-chlorophenyl)-5-(1-methyl-4-piperidinyl)-1 H-pyrazol-4-yl]-Pyrimidine (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Bradykinin Rezeptor Antagonisten gelangen hierbei vorzugsweise Verbindungen zur Anwendung, die ausgewählt sind aus der Gruppe bestehend aus Icatibant und 1-Piperazinepentanaminium, delta-amino-4-[[4-[[[2,4-dichloro-3-[[(2,4-dimethyl-8-chinolinyl)oxy]methyl]phenyl]sulfonyl]amino]tetrahydro-2H-pyran-4-yl]carbonyl]-N,N,N-trimethyl-ε-oxo-, chloride, hydrochloride (1:1:1), (deltaS)- (MEN-16132), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Endothelin Antagonisten gelangen hierbei vorzugsweise Verbindungen zur Anwendung, die ausgewählt sind aus der Gruppe bestehend aus Actelion-1, Ambrisentan, Sitaxsentan, N-(2-acetyl-4,6-dimethylphenyl)-3-[[(4-chloro-3-methyl-5-isoxazolyl)amino]sulfonyl]-2-Thiophenecarboxamid (TBC-3214) und Bosentan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Husten gelangen hierbei vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CXCR1 und /oder CXCR2 Rezeptor Antagonisten gelangen hierbei vorzugsweise Verbindungen zur Anwendung, wie z.B. 3-[[3-[(Dimethylamino)carbonyl]-2-hydroxyphenyl]amino]-4-[[(R)-1-(5-methylfuran-2-yl)propyl]amino]cyclobut-3-ene-1,2-dione (SCH-527123),
gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Erfindungsgemäß bevorzugt sind die Säureadditionssalze der oben genannten Betamimetika, Anticholinergika, Corticosteroide, PDE4 Inhibitoren, LTB4 (BLT1, BLT2) Rezeptor Antagonisten, LTD4 (CysLT1, CysLT2, CysLT3) Rezeptor Antagonisten, Inhibitoren von MAP Kinasen wie zum Beispiel p38, ERK1, ERK2, JNK1, JNK2, JNK3 oder SAP, Bradykinin Rezeptor Antagonisten, Endothelin Rezeptor Antagonisten, Substanzen gegen Husten, CXCR1 und / oder CXCR2 Rezeptor Antagonisten ebenfalls ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

### Pharmazeutische Zusammensetzungen

Die erfindungsgemäßen Verbindung kann oral, transdermal, inhalativ, parenteral oder sublingual verabreicht werden. Die erfindungsgemäße Verbindung liegt hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindung liegt bei einer oralen Anwendung zwischen 0.1 und 5000, vorzugsweise zwischen 1 und 500, besonders bevorzugt zwischen 5-300 mg/Dosis, bei intravenöser, subkutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern, ethanolischen oder wässrigen Lösungen bevorzugt. Gleichfalls ist es möglich, die erfindungsgemäße Verbindung als Infusionslösung, vorzugsweise in einer physiologischen Kochsalzlösung oder Nährsalzlösung einzusetzen.

Die erfindungsgemäße Verbindung kann allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Bei der pharmazeutischen Anwendung wird die erfindungsgemäße Verbindung in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung kann diese beispielsweise mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet werden.

Die nachfolgenden Beispiele illustrieren die vorliegende Erfindung ohne sie jedoch in ihrem Umfang zu beschränken:

### Pharmazeutische Formulierungsbeispiele

### A) Dragees mit 75 mg Wirksubstanz

### Zusammensetzung:

1 Drageekern enthält:

| | |
|---|---|
| Wirksubstanz | 75.0 mg |
| Calciumphosphat | 93.0 mg |
| Maisstärke | 35.5 mg |
| Polyvinylpyrrolidon | 10.0 mg |
| Hydroxypropylmethylcellulose | 15.0 mg |
| Magnesiumstearat | 1.5 mg |
| | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.

| | |
|---|---|
| Drageegewicht: | 245 mg. |

### B) Tabletten mit 100 mg Wirksubstanz

### Zusammensetzung:

1 Tablette enthält:

| | |
|---|---|
| Wirksubstanz | 100.0 mg |
| Milchzucker | 80.0 mg |
| Maisstärke | 34.0 mg |
| Polyvinylpyrrolidon | 4.0 mg |
| Magnesiumstearat | 2.0 mg |
| | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### C) Tabletten mit 150 mg Wirksubstanz

### Zusammensetzung:

1 Tablette enthält:

| | |
|---|---|
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### D) Hartgelatine-Kapseln mit 150 mg Wirksubstanz

### Zusammensetzung:

1 Kapsel enthält:

| | |
|---|---|
| Wirkstoff | 150.0 mg |
| Maisstärke getr. ca. | 180.0 mg |
| Milchzucker pulv. ca. | 87.0 mg |
| Magnesiumstearat | 3.0 mg |
| ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.

Die Endmischung wird in Hartgelatine-Kapseln der Größe 1 abgefüllt.
Kapselfüllung: ca. 320 mg
Kapselhülle: Hartgelatine-Kapsel Größe 1.

### E) Suppositorien mit 150 mg Wirksubstanz

### Zusammensetzung:

1 Zäpfchen enthält:

| | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

### Herstellung:

Nach dem Aufschmelzen der Suppositorienmasse wird der Wirkstoff darin homogen verteilt und die Schmelze in vorgekühlte Formen gegossen.

### F) Suspension mit 50 mg Wirksubstanz

### Zusammensetzung:

100 ml Suspension enthalten:

| | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest. | ad 100.00 ml |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### G) Ampullen mit 10 mg Wirksubstanz

**Zusammensetzung:**

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### H) Ampullen mit 50 mg Wirksubstanz

**Zusammensetzung:**

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. Verfahren zur stereoselektiven Herstellung der Verbindung der Formel **(I)** gegebenenfalls in Form ihrer Tautomeren: 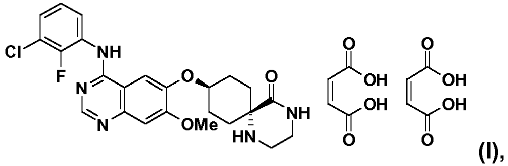 **dadurch gekennzeichnet, dass** das Verfahren die Reaktionsschritte **(A)** bis **(M)** umfasst, wobei
**(A)** die Umsetzung von 1,4-cyclohexandion-mono-ethylenketal zu einer Verbindung der Formel **(1)** 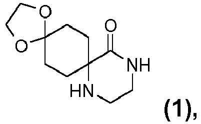
**(B)** die Umsetzung einer Verbindung der Formel **(1)** zu der Verbindung der Formel **(2)** 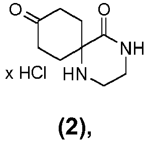
**(C)** die Umsetzung einer Verbindung der Formel **(2)** zu der Verbindung der Formel **(3)** 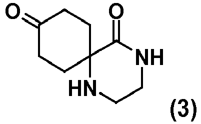
**(D)** die Umsetzung einer Verbindung der Formel **(3)** mit einem Schutzgruppenreagenz zu der Verbindung der Formel **(4)** 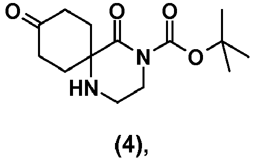
**(E)** die Reduktion einer Verbindung der Formel **(4)** zu der Verbindung der Formel **(5)** 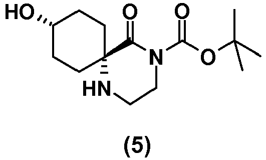
**(F)** die Umsetzung einer Verbindung der Formel **(5)** zu einer Verbindung der Formel **(6)** 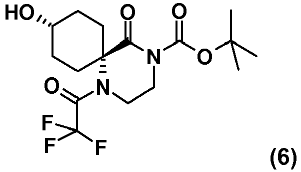
**(G)** die Umsetzung einer Verbindung der Formel **(6)** mit einer Verbindung der Formel **(13)** zu einer Verbindung der Formel **(7)** 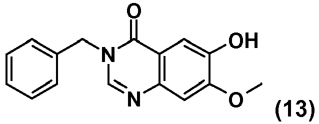 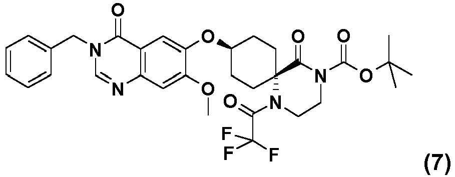
**(H)** die Umsetzung einer Verbindung der Formel **(7)** zu einer Verbindung der Formel **(8a)** oder ihrer tautomeren Form **(8b),** 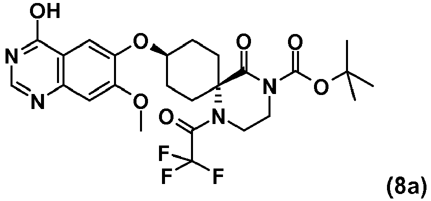 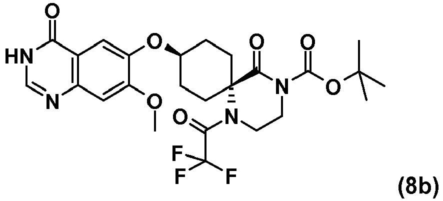
**(I)** die Chlorierung der Verbindung der Formel **(8a)** oder **(8b)** zu einer Verbindung der Formel **(9)** 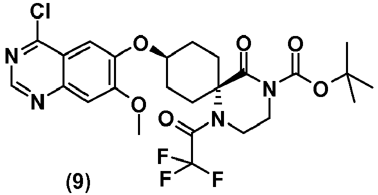
**(J) + (K)** die Umsetzung der Verbindung der Formel **(9)** mit 3-Chlor-2-fluoranilin und Abspaltung der tert-Butylcarbamat-Schutzgruppe zu einer Verbindung der Formel **(11)** oder **(11A)** 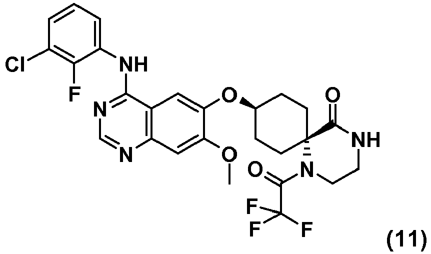 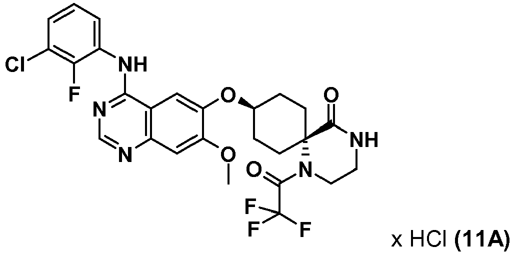
**(L)** Abspaltung der Trifluoracetyl- Schutzgruppe zu der Verbindung der Formel **(II)** 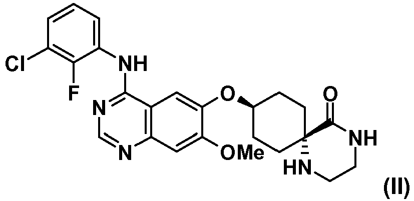
**(M)** Umsetzung der Verbindung der Formel **(II)** mit Maleinsäure zu einer Verbindung der Formel **(I)**, gegebenenfalls in Form ihrer Tautomeren 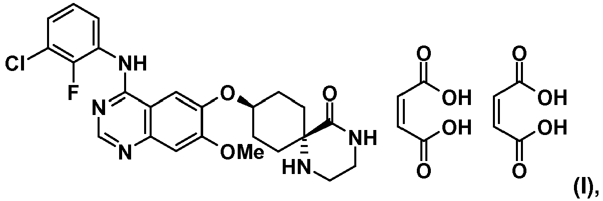 bedeutet,
wobei die Verfahrensschritte **(A)** bis **(M)** in der genannten Reihenfolge nacheinander erfolgen.

2. Verfahren zur stereoselektiven Herstellung der Verbindung der Formel **(I)** gegebenenfalls in Form ihrer Tautomeren: 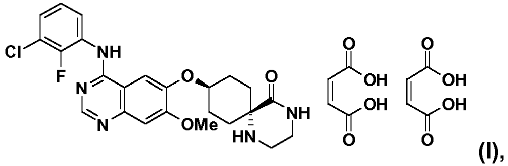 **dadurch gekennzeichnet, dass** das Verfahren die Reaktionsschritte **(A)** bis **(I), (N +O), (L) und (M)** umfasst, wobei
, wobei
**(A)** die Umsetzung von 1,4-cyclohexandion-mono-ethylenketal zu einer Verbindung der Formel **(1)** 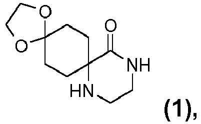
**(B)** die Umsetzung einer Verbindung der Formel **(1)** zu der Verbindung der Formel **(2)** 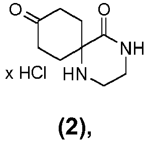
**(C)** die Umsetzung einer Verbindung der Formel **(2)** zu der Verbindung der Formel **(3)** 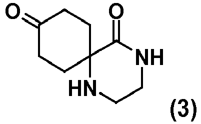
**(D)** die Umsetzung einer Verbindung der Formel **(3)** mit einem Schutzgruppenreagenz zu der Verbindung der Formel **(4)** 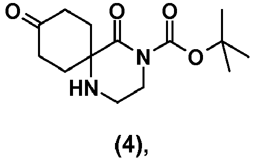
**(E)** die Reduktion einer Verbindung der Formel **(4)** zu der Verbindung der Formel **(5)** 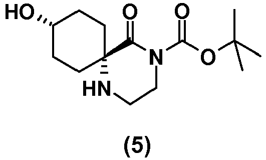
**(F)** die Umsetzung einer Verbindung der Formel **(5)** zu einer Verbindung der Formel **(6)** 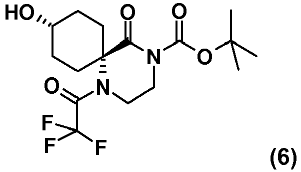
**(G)** die Umsetzung einer Verbindung der Formel **(6)** mit einer Verbindung der Formel **(13)** zu einer Verbindung der Formel **(7)** 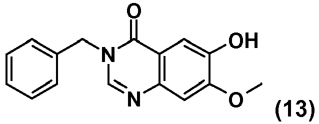 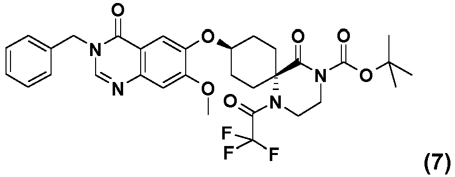
**(H)** die Umsetzung einer Verbindung der Formel **(7)** zu einer Verbindung der Formel **(8a)** oder ihrer tautomeren Form **(8b),** 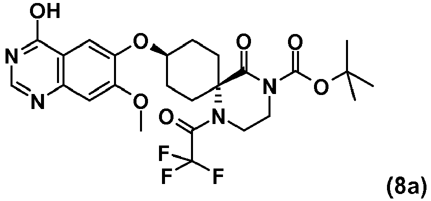 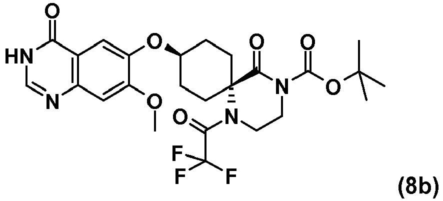
**(I)** die Chlorierung der Verbindung der Formel **(8a)** oder **(8b)** zu einer Verbindung der Formel **(9)** 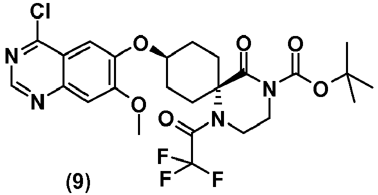
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 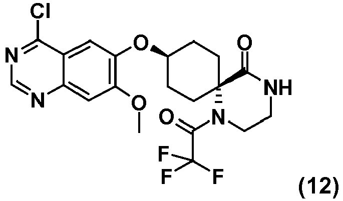
**(L)** Abspaltung der Trifluoracetyl- Schutzgruppe zu der Verbindung der Formel **(II)** 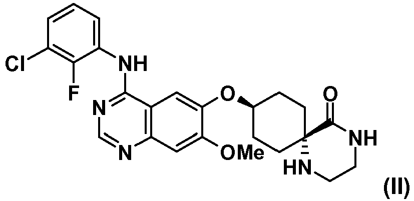
**(M)** Umsetzung der Verbindung der Formel **(II)** mit Maleinsäure zu einer Verbindung der Formel **(I),** gegebenenfalls in Form ihrer Tautomeren 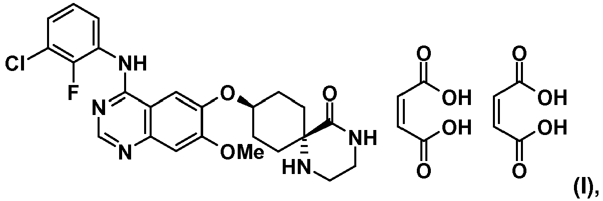 bedeutet,
wobei die Verfahrensschritte **(A)** bis **(I), (N +O), (L) und (M)** in der genannten Reihenfolge nacheinander erfolgen.

3. Verfahren, zur stereoselektiven Herstellung einer Verbindung der Formel **(I), dadurch gekennzeichnet, dass** das Verfahren aus den Verfahrensschritten **(I), (J), (K), (L),** und **(M)** oder aus den Verfahrensschritten **(I),(N), (O), (L)** und **(M)** besteht, wobei
**(I)** die Chlorierung der Verbindung der Formel **(8a)** oder **(8b)** zu einer Verbindung der Formel **(9)** 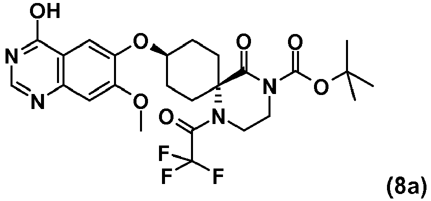 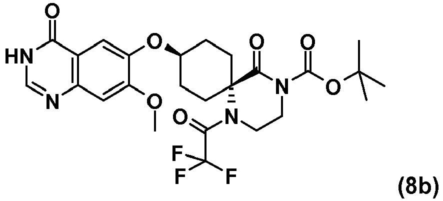 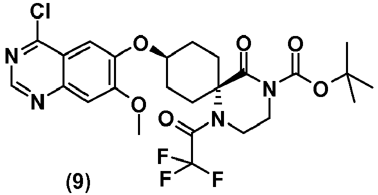
**(J)** + **(K)** die Umsetzung der Verbindung der Formel **(9)** mit 3-Chlor-2-fluoranilin und Abspaltung der tert-Butylcarbamat-Schutzgruppe zu einer Verbindung der Formel **(11)** oder **(11A)** 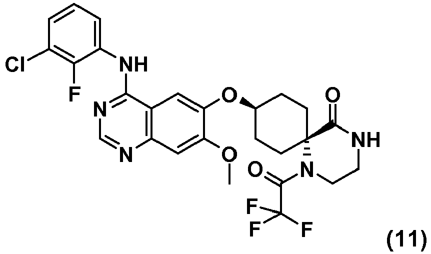 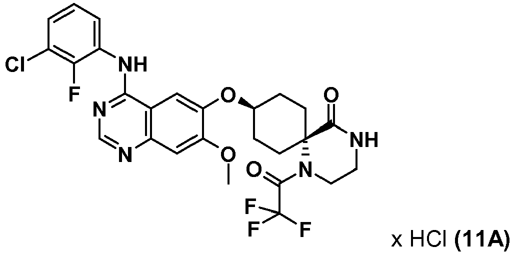
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 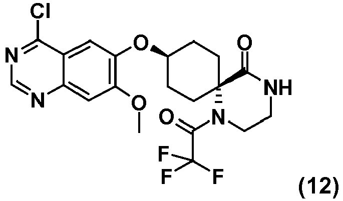
**(L)** Abspaltung der Trifluoracetyl- Schutzgruppe zu der Verbindung der Formel **(II)** 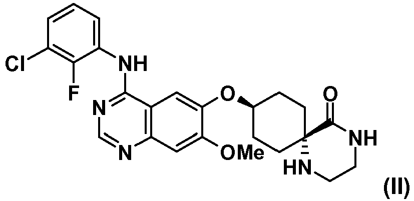
**(M)** Umsetzung der Verbindung der Formel **(II)** mit Maleinsäure zu einer Verbindung der Formel **(I),** gegebenenfalls in Form ihrer Tautomeren 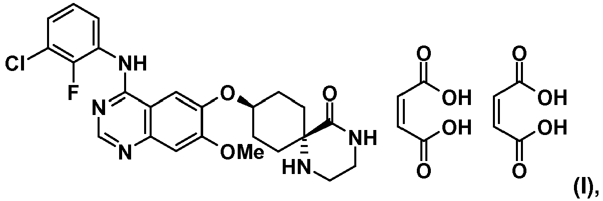 bedeutet,
wobei die Verfahrensschritte **(I), (J), (K), (L),** und **(M)** oder die Verfahrensschritten **(I),(N),**
**(O), (L)** und **(M)** jeweils in der genannten Reihenfolge nacheinander erfolgen.

4. Verfahren zur stereoselektiven Herstellung einer Verbindung der Formel **(II)**, **dadurch gekennzeichnet, dass** das Verfahren aus den Verfahrensschritten **(I), (J), (K)** und **(L),** oder aus den Verfahrensschritten **(I), (N), (O)** und **(L)** besteht, wobei
**(I)** die Chlorierung der Verbindung der Formel **(8a)** oder **(8b)** zu einer Verbindung der Formel **(9)** 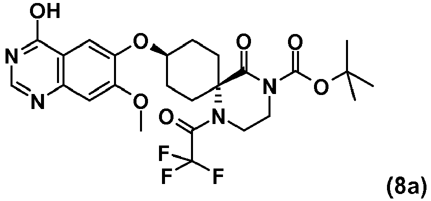 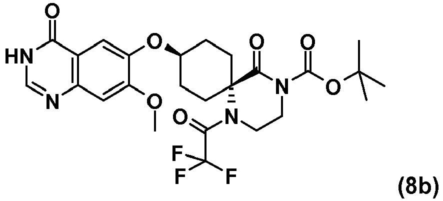 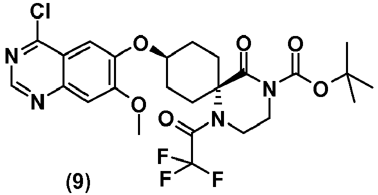
**(J) + (K)** die Umsetzung der Verbindung der Formel **(9)** mit 3-Chlor-2-fluoranilin und Abspaltung der tert-Butylcarbamat-Schutzgruppe zu einer Verbindung der Formel **(11)** oder **(11A)** 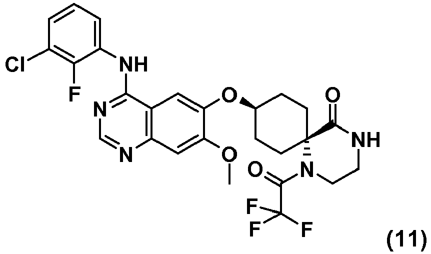 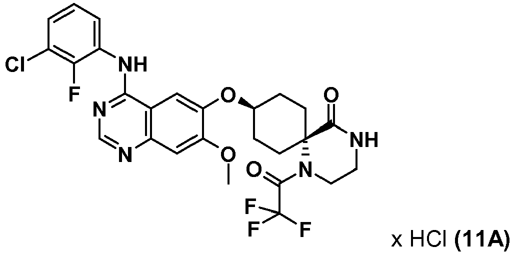
**(N+O)** die Abspaltung der tert-Butylcarbamat-Schutzgruppe der Verbindung der Formel **(9)** unter Bildung der Verbindung der Formel **(12)** und anschließender Umsetzung mit 3-Chlor-2-fluoranilin zur Verbindung der Formel **(11)** oder **(11A)** 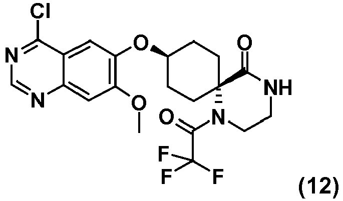
**(L)** Abspaltung der Trifluoracetyl- Schutzgruppe zu der Verbindung der Formel **(II)** 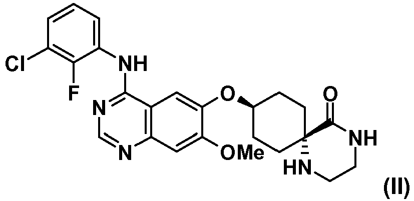 wobei die Verfahrensschritte **(I), (J), (K)** und **(L)** oder die Verfahrensschritten **(I), (N), (O)** und **(L)** jeweils in der genannten Reihenfolge nacheinander erfolgen.

5. Verfahrensschritt **(G), dadurch gekennzeichnet, dass**
**(G)** die Umsetzung einer Verbindung der Formel **(6)** mit einer Verbindung der Formel **(13)** zu einer Verbindung der Formel **(7)** 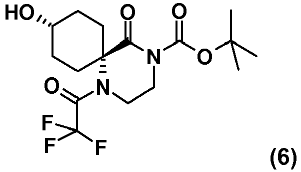 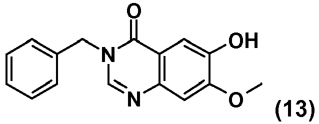 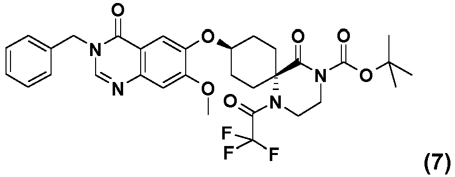 bedeutet.

6. Verfahrensschritt **(I)**, **dadurch gekennzeichnet, dass**
**(I)** die Chlorierung der Verbindung der Formel **(8a)** oder **(8b)** zu einer Verbindung der Formel **(9)** 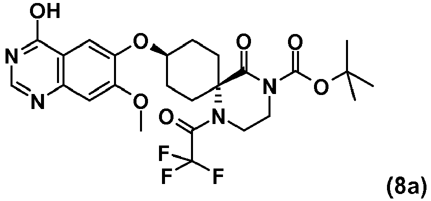 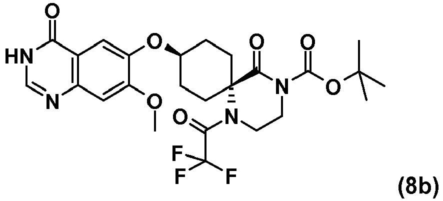 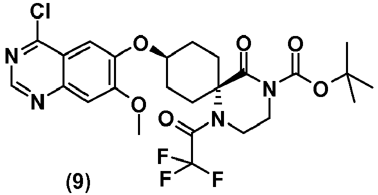 bedeutet.

7. Intermediat der Formel **(6)**, gegebenenfalls in Form ihrer Tautomeren.

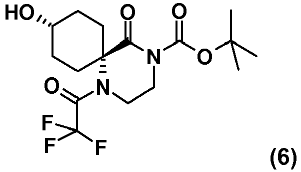

8. Intermediat der Formel **(7)**, gegebenenfalls in Form ihrer Tautomeren.

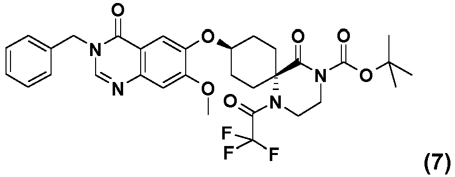

9. Intermediat der Formel **(8a)** oder **(8b)**, gegebenenfalls in Form ihrer Tautomeren.

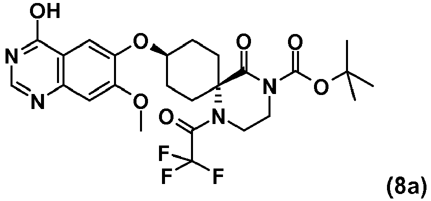

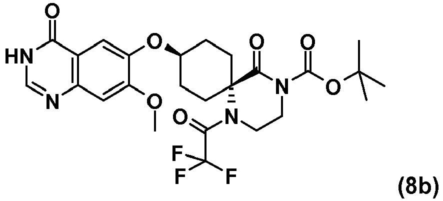

10. Intermediat der Formel **(9)**, gegebenenfalls in Form ihrer Tautomeren.

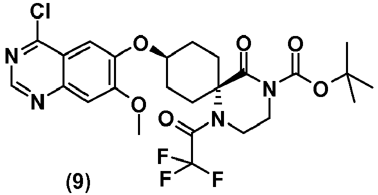

11. Intermediat der Formel **(11)** oder **(11A),** gegebenenfalls in Form ihrer Tautomeren.

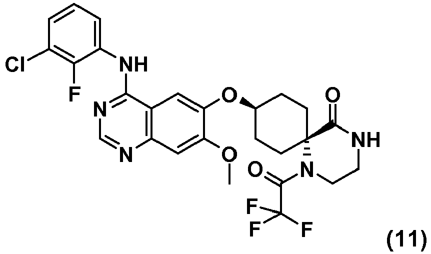

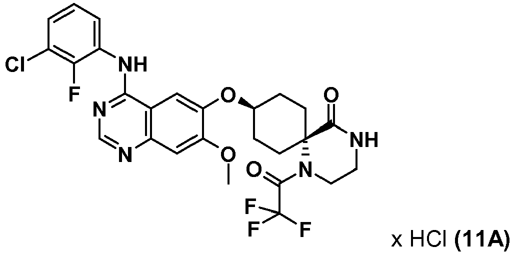

## Claims

1. Process for the stereoselective preparation of the compound of formula **(I)**, optionally in the form of the tautomers thereof: 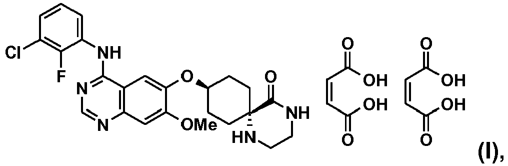 **characterised in that** the process comprises reaction steps **(A)** to **(M)**, wherein
**(A)** is the reaction of 1,4-cyclohexanedione-mono-ethyleneketal to form a compound of formula **(1)** 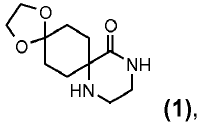
**(B)** is the reaction of a compound of formula **(1)** to form the compound of formula **(2)** 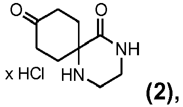
**(C)** is the reaction of a compound of formula **(2)** to form the compound of formula **(3)** 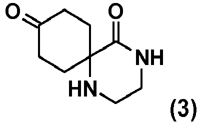
**(D)** is the reaction of a compound of formula **(3)** with a protective group reagent to form the compound of formula **(4)** 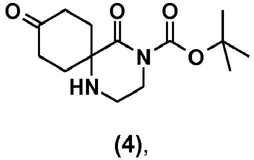
**(E)** is the reduction of a compound of formula **(4)** to form the compound of formula **(5)** 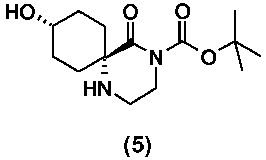
**(F)** is the reaction of a compound of formula **(5)** to form a compound of formula **(6)** 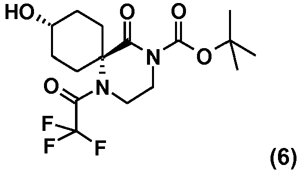
**(G)** is the reaction of a compound of formula **(6)** with a compound of formula **(13)** to form a compound of formula **(7)** 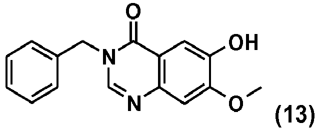 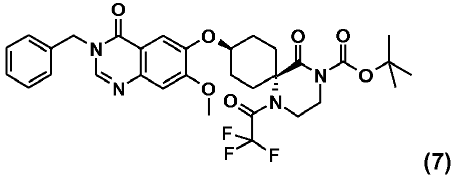
**(H)** is the reaction of a compound of formula **(7)** to form a compound of formula **(8a)** or its tautomeric form **(8b)**, 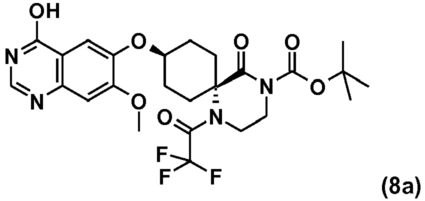 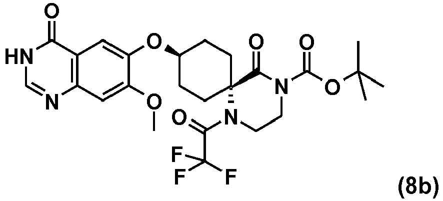
**(I)** is the chlorination of the compound of formula **(8a)** or **(8b)** to form a compound of formula **(9)** 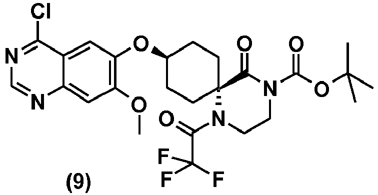
**(J) + (K)** is the reaction of the compound of formula **(9)** with 3-chloro-2-fluoroaniline and the cleaving of the tert-butyl carbamate protective group to form a compound of formula **(11)** or **(11A)** 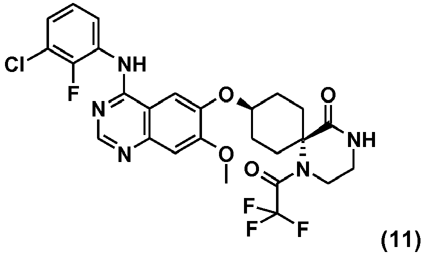 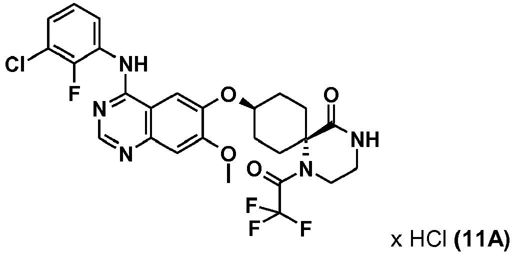
**(L)** is the cleaving of the trifluoroacetyl protective group to form the compound of formula **(II)** 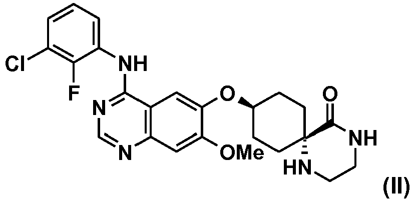
**(M)** is the reaction of the compound of formula **(II)** with maleic acid to form a compound of formula **(I)**, optionally in the form of the tautomers thereof 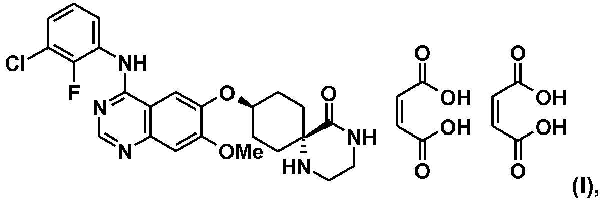 wherein process steps **(A)** to **(M)** take place successively in the sequence specified.

2. Process for the stereoselective preparation of the compound of formula **(I)**, optionally in the form of the tautomers thereof: 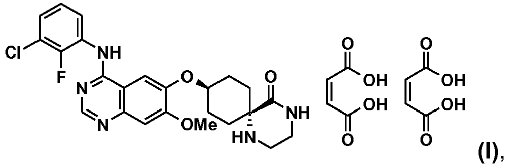 **characterised in that** the process comprises reaction steps **(A)** to **(I)**, **(N+O), (L)** and **(M)**, wherein
**(A)** is the reaction of 1,4-cyclohexanedione-mono-ethyleneketal to form a compound of formula **(1)** 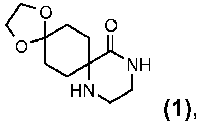
**(B)** is the reaction of a compound of formula **(1)** to form the compound of formula **(2)** 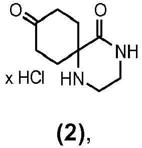
**(C)** is the reaction of a compound of formula **(2)** to form the compound of formula **(3)** 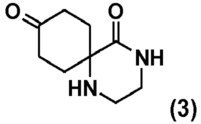
**(D)** is the reaction of a compound of formula **(3)** with a protective group reagent to form the compound of formula **(4)** 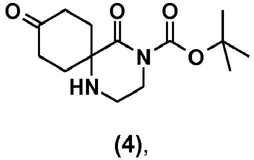
**(E)** is the reduction of a compound of formula **(4)** to form the compound of formula **(5)** 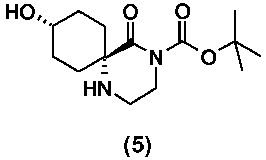
**(F)** is the reaction of a compound of formula **(5)** to form a compound of formula **(6)** 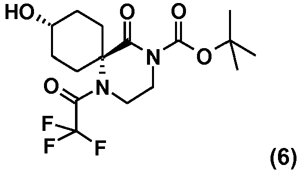
**(G)** is the reaction of a compound of formula **(6)** with a compound of formula **(13)** to form a compound of formula **(7)** 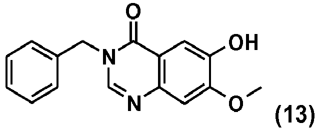 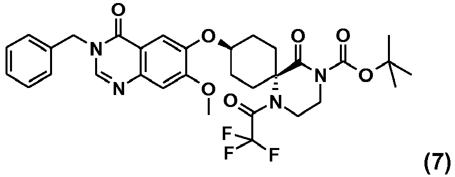
**(H)** is the reaction of a compound of formula **(7)** to form a compound of formula **(8a)** or its tautomeric form **(8b),** 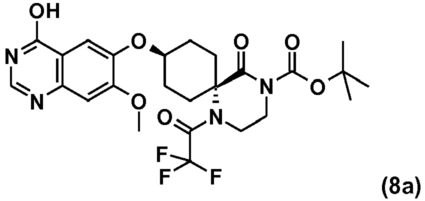 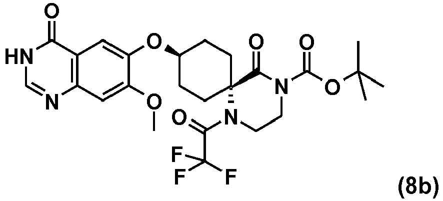
**(I)** is the chlorination of the compound of formula **(8a)** or **(8b)** to form a compound of formula **(9)** 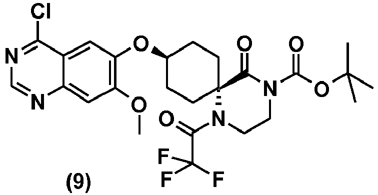
**(N+O)** is the cleaving of the tert-butyl carbamate protective group of the compound of formula **(9)** to form the compound of formula **(12)** and the subsequent reaction with 3-chloro-2-fluoroaniline to form the compound of formula **(11)** or **(11A)** 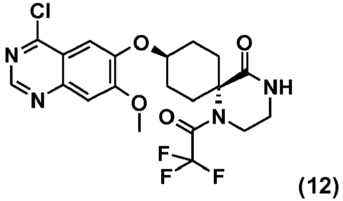
**(L)** is the cleaving of the trifluoroacetyl protective group to form the compound of formula **(II)** 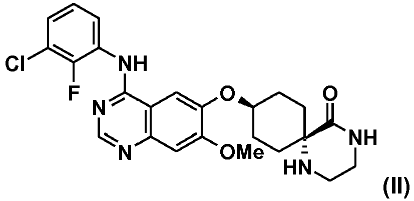
**(M)** is the reaction of the compound of formula **(II)** with maleic acid to form a compound of formula **(I),** optionally in the form of the tautomers thereof 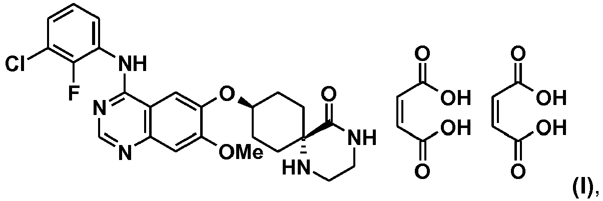 wherein process steps **(A)** to **(I)**, **(N + O), (L)** and **(M)** take place successively in the sequence specified.

3. Process for the stereoselective preparation of a compound of formula **(I), characterised in that** the process consists of process steps **(I)**, **(J)**, **(K)**, **(L)** and **(M)** or of process steps **(I)**, **(N)**, **(O)**, **(L)** and **(M),** wherein
**(I)** is the chlorination of the compound of formula **(8a)** or **(8b)** to form a compound of formula **(9)** 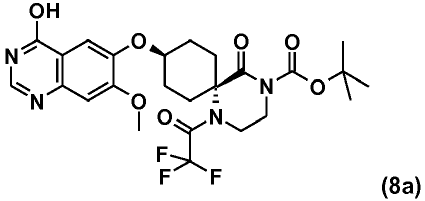 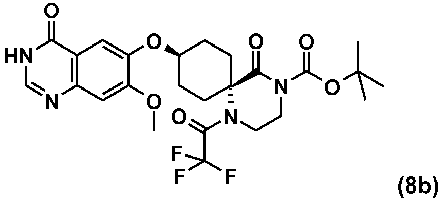 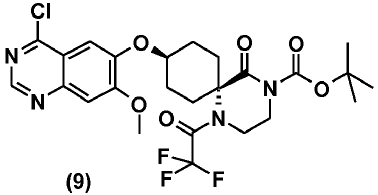
**(J) + (K)** are the reaction of the compound of formula **(9)** with 3-chloro-2-fluoraniline and the cleaving of the tert-butyl carbamate protective group to form a compound of formula **(11)** or **(11A)** 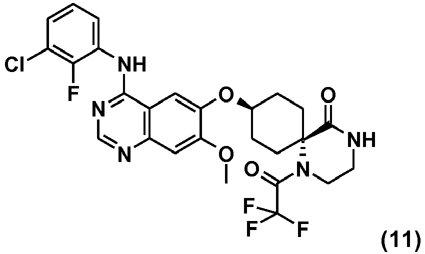 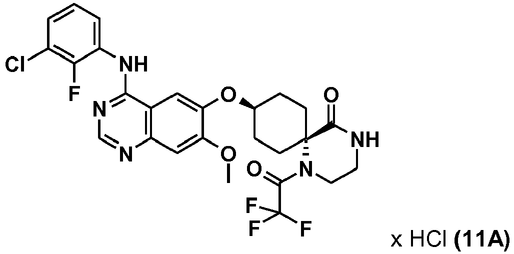
**(N+O)** is the cleaving of the tert-butyl carbamate protective group of the compound of formula **(9)** to form the compound of formula **(12)** and the subsequent reaction with 3-chloro-2-fluoroaniline to form the compound of formula **(11)** or **(11A)** 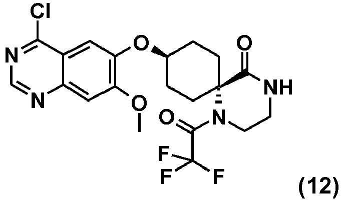
**(L)** is the cleaving of the trifluoroacetyl protective group to form the compound of formula **(II)** 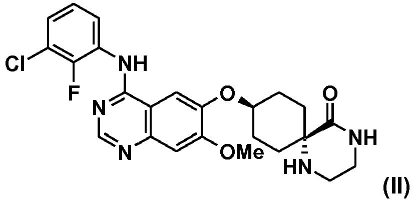
**(M)** is the reaction of the compound of formula **(II)** with maleic acid to form a compound of formula **(I)**, optionally in the form of the tautomers thereof 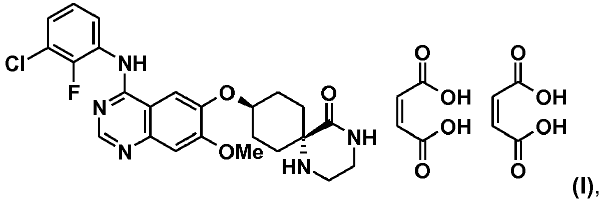 wherein process steps **(I), (J), (K), (L)** and **(M)** or process steps **(I), (N), (O), (L)** and **(M)** in each case take place successively in the sequence specified.

4. Process for the stereoselective preparation of a compound of formula **(II), characterised in that** the process consists of process steps **(I), (J), (K)** and **(L)** or of process steps **(I), (N), (O)** and **(L),** wherein
**(I)** is the chlorination of the compound of formula **(8a)** or **(8b)** to form a compound of formula **(9)** 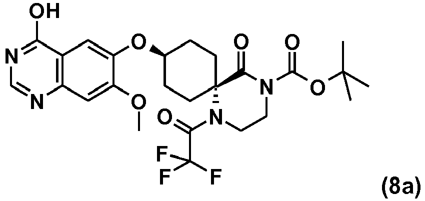 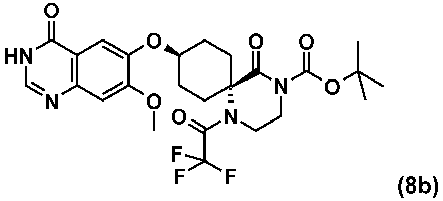 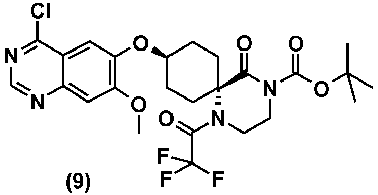
**(J) + (K)** is the reaction of the compound of formula **(9)** with 3-chloro-2-fluoroaniline and the cleaving of the tert-butyl carbamate protective group to form a compound of formula **(11)** or **(11A)** 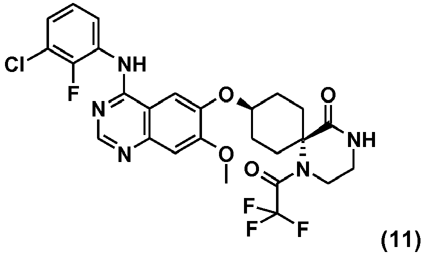 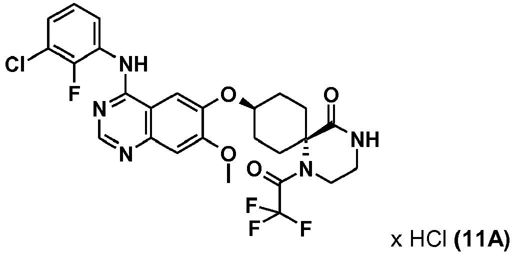
**(N+O)** is the cleaving of the tert-butyl carbamate protective group of the compound of formula **(9)** to form the compound of formula **(12)** and the subsequent reaction with 3-chloro-2-fluoroaniline to form the compound of formula **(11)** or **(11A)** 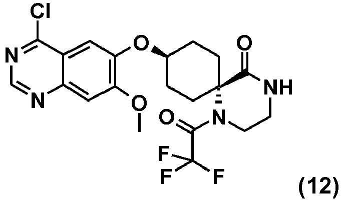
**(L)** is the cleaving of the trifluoroacetyl protective group to form the compound of formula **(II)** 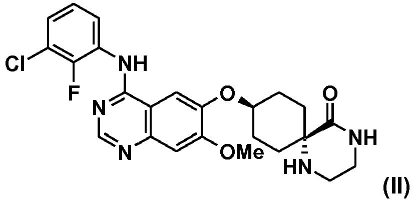 wherein process steps **(I)**, **(J)**, **(K)** and **(L)** or process steps **(I)**, **(N)**, **(O)** and **(L)** in each case take place successively in the sequence specified.

5. Process step **(G)**, **characterised in that**
**(G)** is the reaction of a compound of formula **(6)** with a compound of formula **(13)** to form a compound of formula **(7)**

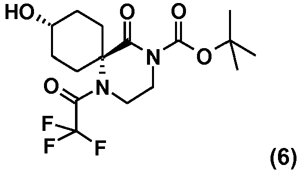

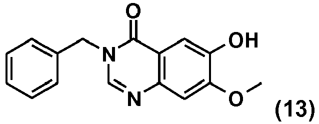

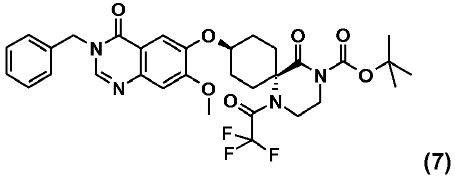

6. Process step **(I)**, **characterised in that**
**(I)** is the chlorination of the compound of formula **(8a)** or **(8b)** to form a compound of formula **(9)**

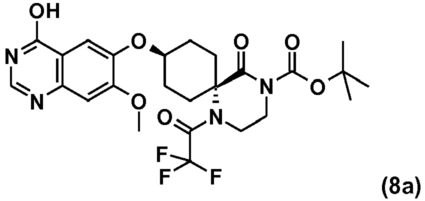

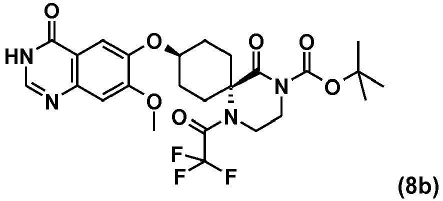

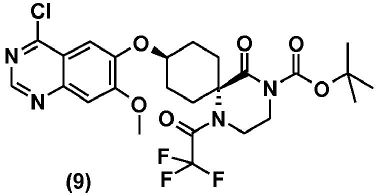

7. Intermediate of formula **(6)**, optionally in the form of the tautomers thereof.

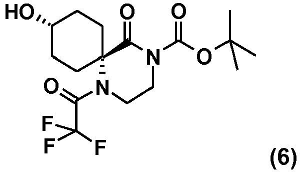

8. Intermediate of formula **(7)**, optionally in the form of the tautomers thereof.

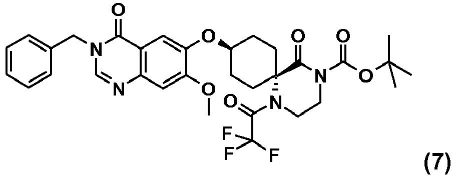

9. Intermediate of formula **(8a)** or **(8b),** optionally in the form of the tautomers thereof.

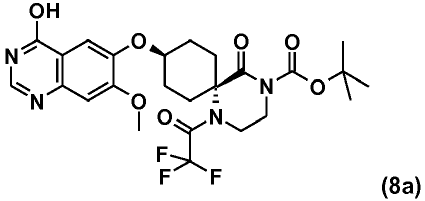

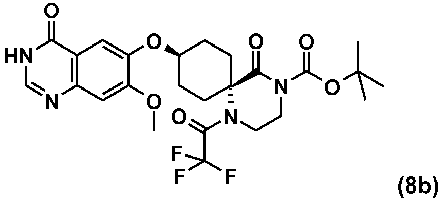

10. Intermediate of formula **(9)**, optionally in the form of the tautomers thereof.

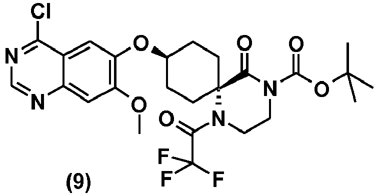

11. Intermediate of formula **(11)** or **(11A)**, optionally in the form of the tautomers thereof.

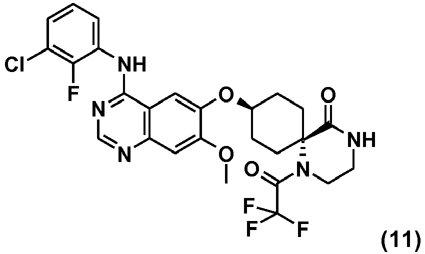

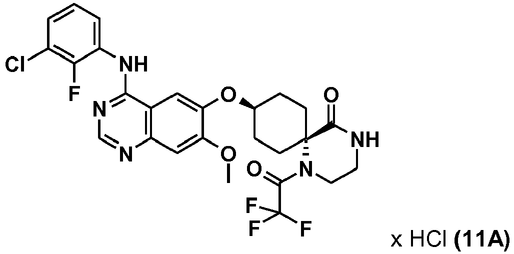

## Revendications

1. Procédé de fabrication stéréosélective du composé de formule **(I)**, le cas échéant sous la forme de ses tautomères : 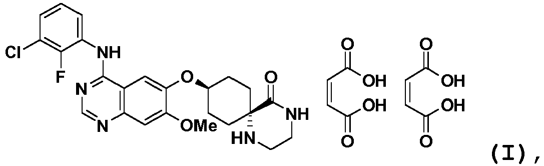 **caractérisé en ce qu'**il comporte les étapes réactionnelles **(A)** à **(M)** suivantes
**(A)** consistant à convertir le mono-éthylène cétal de 1,4-cyclohexanedione en un composé de formule **(1)** 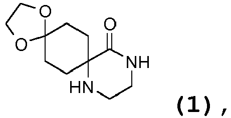
**(B)** consistant à convertir un composé de formule **(1)** en composé de formule **(2)** 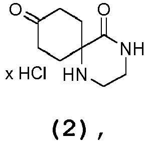
**(C)** consistant à convertir un composé de formule **(2)** en composé de formule **(3)** 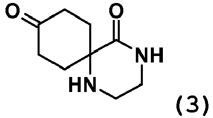
**(D)** consistant à convertir un composé de formule **(3)**, avec un réactif de groupe protecteur, en composé de formule **(4)** 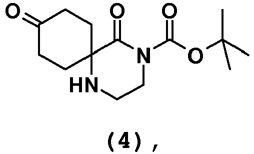
**(E)** consistant à réduire un composé de formule **(4)** en composé de formule **(5)** 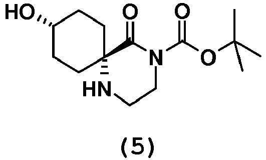
**(F)** consistant à convertir un composé de formule **(5)** en composé de formule **(6)** 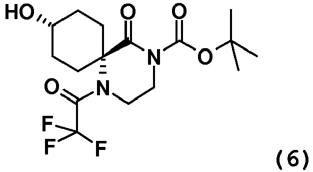
**(G)** consistant à convertir un composé de formule **(6)** avec un composé de formule **(13)** en composé de formule **(7)** 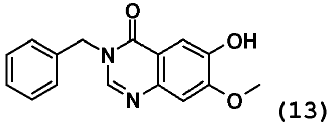 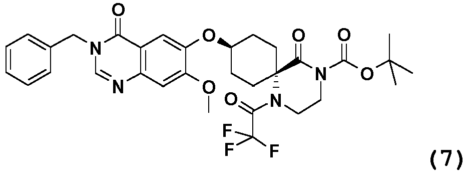
**(H)** consistant à convertir un composé de formule **(7)** en composé de formule **(8a)** ou sa forme tautomère **(8b),** 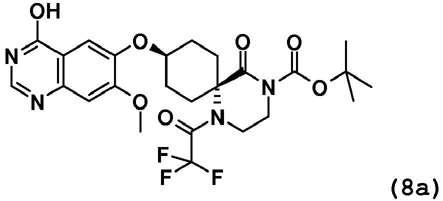 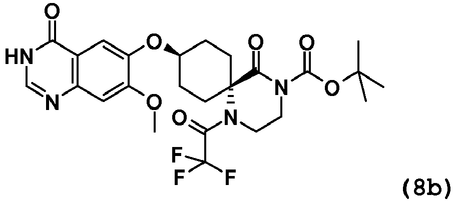
**(I)** consistant à chlorer le composé de formule **(8a)** ou **(8b)** en composé de formule **(9)** 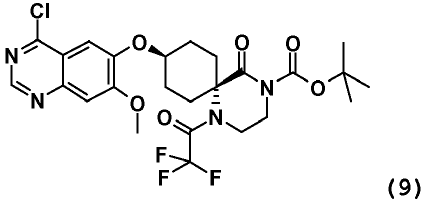
**(J)** + **(K)** consistant à convertir le composé de formule **(9)** avec de la 3-chloro-2-fluoraniline et à cliver le groupe protecteur tert-butylcarbamate en composé de formule **(11)** ou **(11A)** 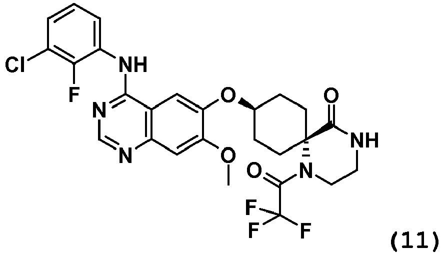 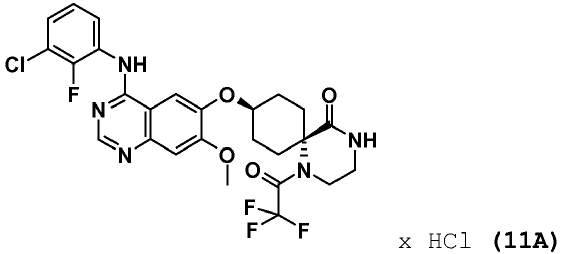
**(L)** consistant à cliver le groupe protecteur trifluoroacétyle en composé de formule **(II)** 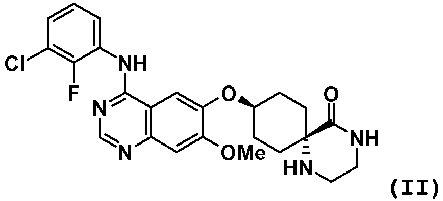
**(M)** consistant à convertir le composé de formule **(II)**, avec de l'acide maléique, en composé de formule **(I)**, le cas échéant sous la forme de ses tautomères 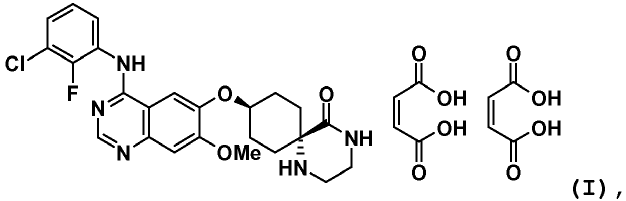 les étapes **(A)** à **(M)** se succédant dans l'ordre indiqué.

2. Procédé de fabrication stéréosélective du composé de formule **(I)**, le cas échéant sous la forme de ses tautomères : 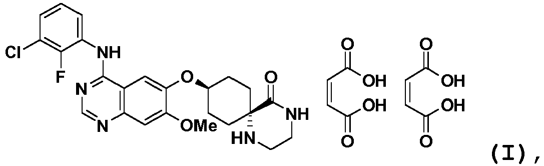 **caractérisé en ce qu'**il comporte les étapes réactionnelles **(A)** à **(I)**, **(N+O)**, **(L)** et **(M)** suivantes
**(A)** consistant à convertir le mono-éthylène cétal de 1,4-cyclohexanedione en un composé de formule **(1)** 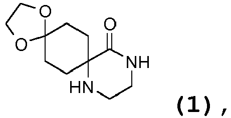
**(B)** consistant à convertir un composé de formule **(1)** en composé de formule **(2)** 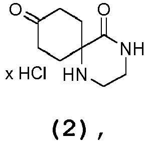
**(C)** consistant à convertir un composé de formule **(2)** en composé de formule **(3)** 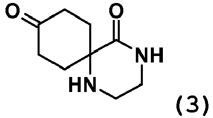
**(D)** consistant à convertir un composé de formule **(3)**, avec un réactif de groupe protecteur, en composé de formule **(4)** 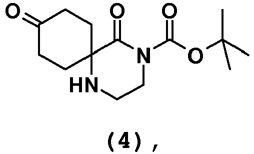
**(E)** consistant à réduire un composé de formule **(4)** en composé de formule **(5)** 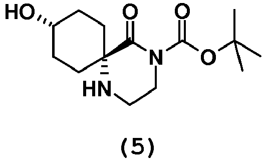
**(F)** consistant à convertir un composé de formule **(5)** en composé de formule **(6)** 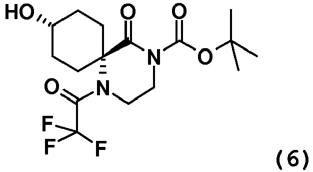
**(G)** consistant à convertir un composé de formule **(6)** avec un composé de formule **(13)** en composé de formule **(7)** 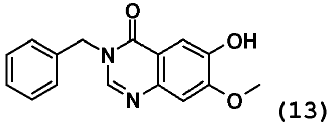 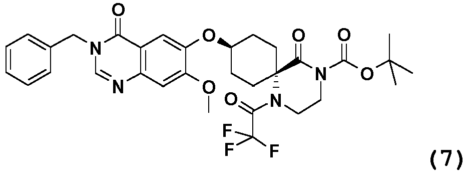
**(H)** consistant à convertir un composé de formule **(7)** en composé de formule **(8a)** ou sa forme tautomère **(8b),** 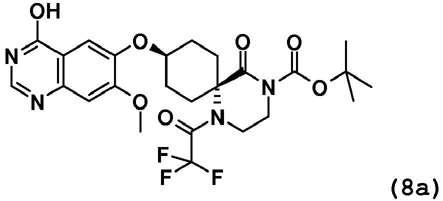 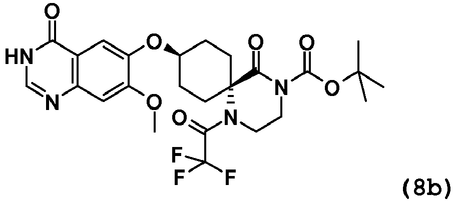
**(I)** consistant à chlorer le composé de formule **(8a)** ou **(8b)** en composé de formule **(9)** 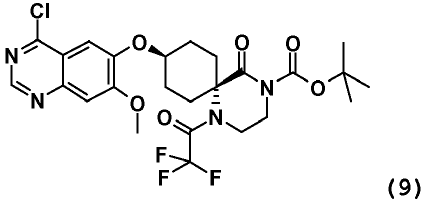
**(N + O)** consistant à cliver le groupe protecteur tert-butylcarbamate du composé de formule **(9)** en formant le composé de formule **(12)** et à le convertir finalement avec de la 3-chloro-2-fluoraniline en composé de formule **(11)** ou **(11A)** 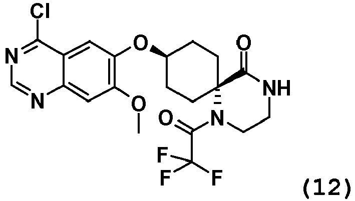
**(L)** consistant à cliver le groupe protecteur trifluoroacétyle en composé de formule **(II)** 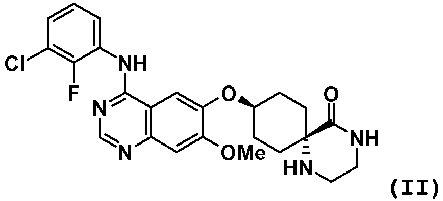
**(M)** consistant à convertir le composé de formule **(II)**, avec de l'acide maléique, en composé de formule **(I)**, le cas échéant sous la forme de ses tautomères 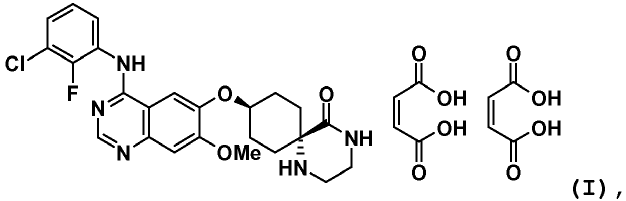 les étapes **(A)** à **(I)**, **(N+O), (L)** et **(M)** se succédant dans l'ordre indiqué.

3. Procédé de fabrication stéréosélective d'un composé de formule **(I)**, **caractérisé en ce qu'**il comporte les étapes réactionnelles **(I), (J), (K), (L)** et **(M)** suivantes ou les étapes réactionnelles **(I), (N)**, **(O), (L)** et **(M)** suivantes
**(I)** consistant à chlorer le composé de formule **(8a)** ou **(8b)** en composé de formule **(9)** 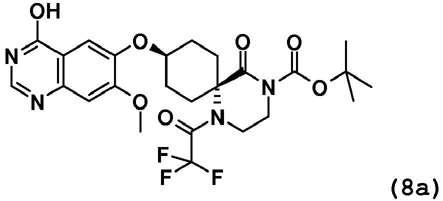 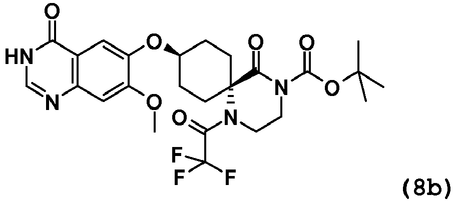 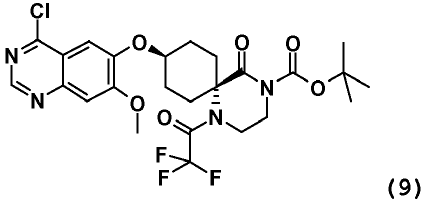
**(J) + (K)** consistant à convertir le composé de formule **(9)** avec de la 3-chloro-2-fluoraniline et à cliver le groupe protecteur tert-butylcarbamate en composé de formule **(11)** ou **(11A)** 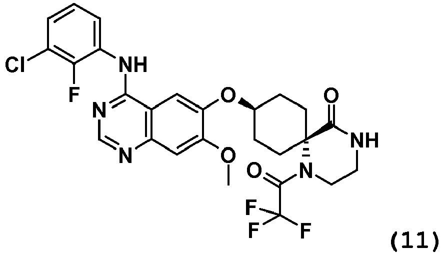 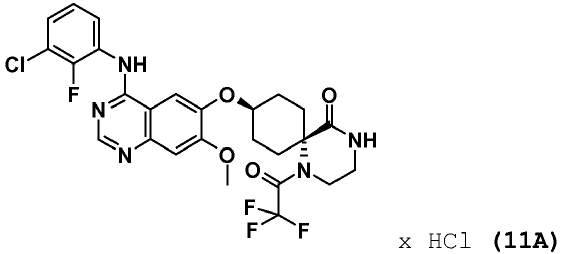
**(N + O)** consistant à cliver le groupe protecteur tert-butylcarbamate du composé de formule **(9)** en formant le composé de formule **(12)** et à le convertir finalement avec de la 3-chloro-2-fluoraniline en composé de formule **(11)** ou **(11A)** 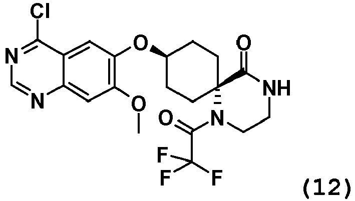
**(L)** consistant à cliver le groupe protecteur trifluoroacétyle en composé de formule **(II)** 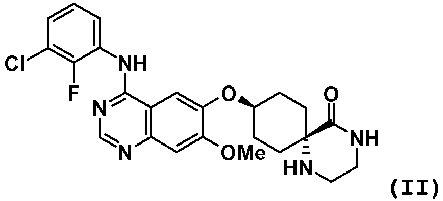
**(M)** consistant à convertir le composé de formule **(II)**, avec de l'acide maléique, en composé de formule **(I)**, le cas échéant sous la forme de ses tautomères 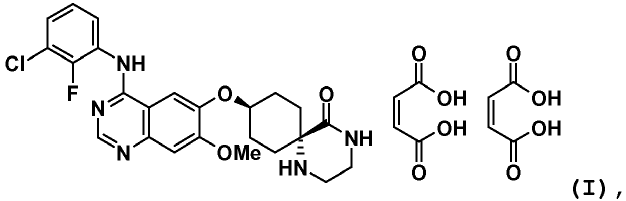 les étapes **(I), (J), (K), (L)** et **(M)** ou les étapes **(I), (N)**, **(O)**, **(L)** et **(M)** se succédant respectivement dans l'ordre indiqué.

4. Procédé de fabrication stéréosélective d'un composé de formule **(II), caractérisé en ce qu'**il comporte les étapes réactionnelles **(I), (J), (K)** et **(L)** suivantes ou les étapes réactionnelles **(I), (N), (O)** et **(L)** suivantes
**(I)** consistant à chlorer le composé de formule **(8a)** ou **(8b)** en composé de formule **(9)** 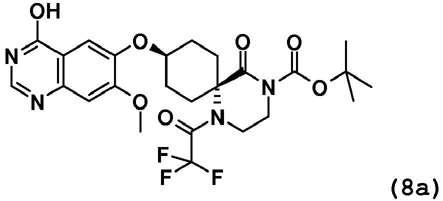 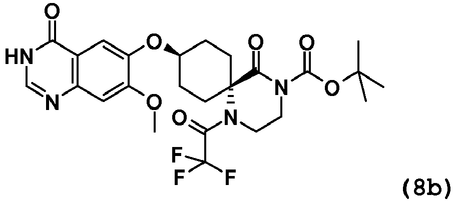 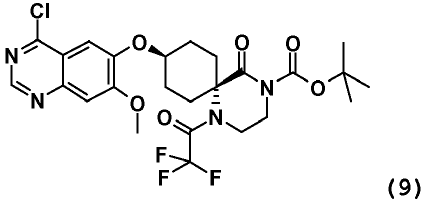
**(J) + (K)** consistant à convertir le composé de formule **(9)** avec de la 3-chloro-2-fluoraniline et à cliver le groupe protecteur tert-butylcarbamate en composé de formule **(11)** ou **(11A)** 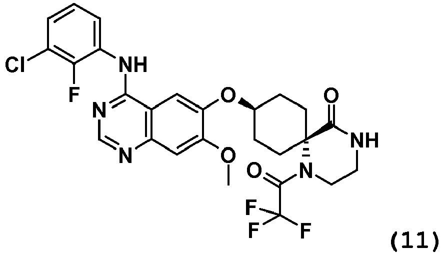 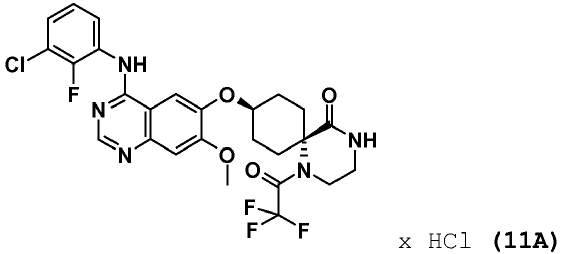
**(N + O)** consistant à cliver le groupe protecteur tert-butylcarbamate du composé de formule **(9)** en formant le composé de formule **(12)** et à le convertir finalement avec de la 3-chloro-2-fluoraniline en composé de formule **(11)** ou **(11A)** 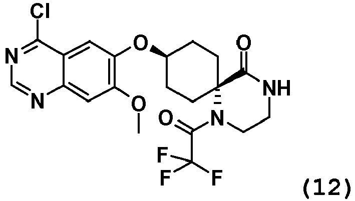
**(L)** consistant à cliver le groupe protecteur trifluoroacétyle en composé de formule **(II)** 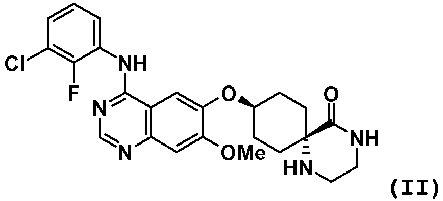 les étapes **(I), (J), (K)** et **(L)** ou les étapes **(I), (N), (O),** et **(L)** se succédant respectivement dans l'ordre indiqué.

5. Etape réactionnelle **(G), caractérisée en ce qu'**elle
**(G)** consiste à convertir un composé de formule **(6)** avec un composé de formule **(13)** en composé de formule **(7)**

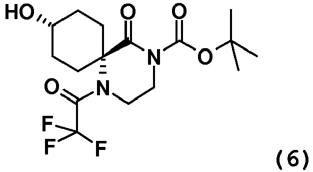

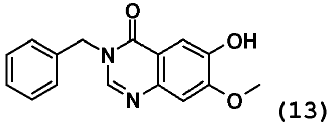

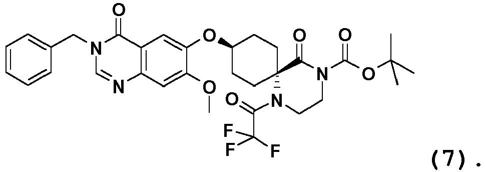

6. Etape réactionnelle **(I), caractérisée en ce qu'**elle
**(I)** consiste à chlorer le composé de formule **(8a)** ou **(8b)** en composé de formule **(9)**

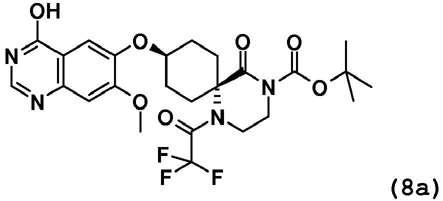

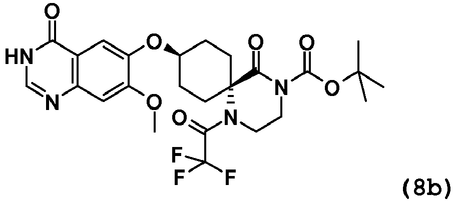

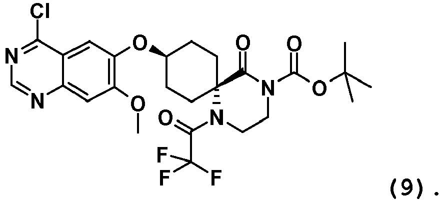

7. Intermédiaire de formule **(6),** le cas échéant sous la forme de ses tautomères.

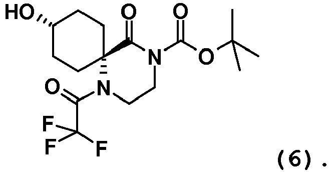

8. Intermédiaire de formule **(7),** le cas échéant sous la forme de ses tautomères.

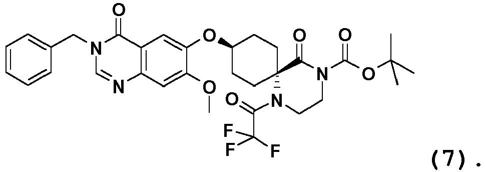

9. Intermédiaire de formule **(8a)** ou **(8b),** le cas échéant sous la forme de ses tautomères.

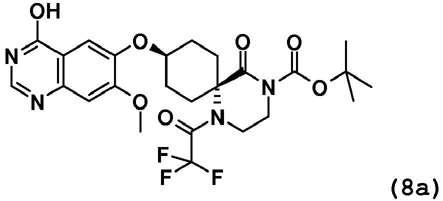

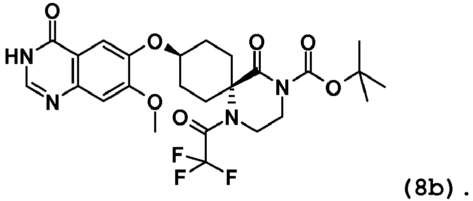

10. Intermédiaire de formule **(9),** le cas échéant sous la forme de ses tautomères.

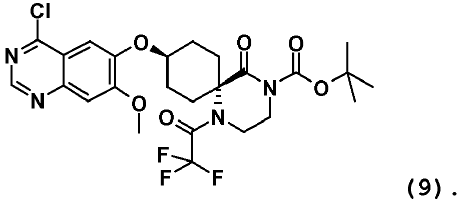

11. Intermédiaire de formule **(11)** ou **(11A),** le cas échéant sous la forme de ses tautomères.
